# EUROPEAN PATENT APPLICATION

(11) **EP 4 834 198 A1**
(43) Date of publication of application: **04.11.2026**
(21) Application number: 26205954.6
(22) Date of filing: 20.01.2020
(51) Int. Cl.: A61B 1/015, A61B 1/00, A61B 1/005, A61B 1/05, A61B 1/018, A61B 18/26, A61B 18/00, A61B 18/22, A61B 90/00, A61B 90/30

(54) **EFFICIENT MULTI-FUNCTIONAL ENDOSCOPIC INSTRUMENT**

(30) Priority: 18.01.2019 US 201962794328 P
(62) Document number(s) of the earlier application(s) in accordance with Art. 76 EPC: 20741363.4 / 3 911 258
(71) Applicant: IPG Photonics Corporation, Oxford, MA 01540 (US)
(72) Inventor: ALTSHULER, Gregory, Lincoln, 01773 (US); YAROSLAVSKY, Ilya, N. Andover, 01845 (US); BOUTOUSSOV, Dmitri, Southborough, 01772 (US); ANDREEVA, Viktoriya, 141006 Mytishchi (RU); KOVALENKO, Anastasiya, 141006 Mytishchi (RU); TRAXER, Olivier, 75020 Paris (FR); BARENBOYM, Michael, Boston, 02118 (US); OSTROVSKY, Isaac, Wellesley, 02481 (US)
(74) Representative: Peterreins Schley

(57) **Abstract**

The present disclosure relates to an endoscopic surgical instrument, comprising: a catheter shaft defining and extending along a central axis and including a proximal portion and a distal portion; a distal head portion disposed at said distal portion of said catheter shaft, said distal head portion including a distal face; a working channel extending within said catheter shaft from said proximal portion through said distal head portion, said distal head portion defining a mouth at said distal face, said working channel being configured to receive a laser fiber; an illuminator disposed at said distal head portion; and an imaging receiver disposed at said distal head portion, said imaging receiver being positioned at an axial distance proximal to a distal extremity of said distal face, said axial distance being within a range of 1 millimeter to 10 millimeters inclusive, wherein said mouth is at least partially within a viewing angle of said imaging receiver.

## Description

### RELATED APPLICATIONS

This application claims the benefit of U.S. Provisional Patent Application No. 62/794,328, filed January 18, 2019, the disclosure of which is hereby incorporated by reference herein in their entirety.

### FIELD OF THE DISCLOSURE

This application is directed generally to endoscopic devices and methods. More specifically, this application is directed to flexible, semi rigid, and rigid laser endoscopes for laser treatment of stones and tissues in humans and animals.

### BACKGROUND OF THE DISCLOSURE

Kidney stones affect 1 in 500 Americans each year, causing significant pain and healthcare expense. Surgical options for patients with symptomatic kidney stones include extracorporeal shock wave lithotripsy (ESWL), ureteroscopy, and percutaneous nephrolithotomy (PCNL). A person's renal anatomy, stone composition, and body habitus all play major roles in determining outcomes and operative approach.

The role of ureteroscopy over the last ten years has increased due to reductions in the diameter of the flexible catheter shaft, enhanced steering and deflection capabilities, improvement of video-imaging, miniaturization of baskets and instruments, and advances in lithotripsy (stone breakage) with the advent of holmium (Ho) and thulium (Tm) lasers. Over 45% of all kidney stone surgeries in the United States are now done using small ureteroscope technology and laser.

Ureteroscopy involves the use of a small flexible or rigid device called a ureteroscope to directly see and treat kidney stones. The ureteroscope device, which provides a video image and has small "working" channels, is inserted into the bladder and up the ureter until the kidney stone is encountered. The kidney stone can then either be broken up with laser energy that is transmitted via a fiber optic (laser fiber) to the target site, and/or extracted using small baskets. The advantage of this type of surgery is that body orifices are used for access, requiring no incisions.

Ureteroscopy is often a good option for small kidney stones in the ureter or kidney. Success rates for ureteroscopy for clearing smaller kidney stones is generally higher than that for shockwave lithotripsy. With laser ureteroscopy, kidney stones can be broken into small particles with maximum dimensions less than 1 millimeter or even less than 0.25 millimeter using laser settings optimized for the purpose. In this case, products of ablation can be removed with irrigation flows or after surgery due to natural outflow from kidney to bladder to provide stone free treatment results.

However, ureteroscopy does not always work well with very large kidney stones (e.g., with dimensions greater than 20 millimeters), as the large size necessitates long treatment times and can pose difficulties in removing the fragments of such stones. Furthermore, mid-sized stones or fragments (e.g., with maximum dimensions of 1 to 5 millimeters) can be difficult to treat with lasers using contact techniques. For example, ureteroscopes operating in contact mode can be subject to strong retropulsion effects, thereby requiring operation in a non-contact mode (e.g., "popcorning"), which is time consuming and does not guarantee stone free results. As a result, ureteroscopy does not always work well with very large kidney stones, as the large size necessitates long treatment time and can pose difficulties in removing the fragments of such stones. In such cases a percutaneous approach may be the best available option. A device and attendant techniques that mitigate or resolve these disadvantages of ureteroscopy would be welcomed.

### SUMMARY OF THE DISCLOSURE

Various embodiments of the disclosure present endoscopic surgical instruments and methods that mitigate certain shortcomings of conventional ureteroscopy while decreasing the treatment time, providing a higher probability of stone free results, and increasing the safety of the treatment.

Conventional ureteroscopes include a working channel that passes through the catheter shaft and defines an inlet at a distal end. The primary functions of the working channel are to serve as conduit for laser fiber optics as well as for other instruments and to deliver irrigation flow. Some conventional ureteroscopes utilize an input face of the imaging assembly that lies essentially on or very close to the same plane as the distal opening of the working channel. Other conventional ureteroscopes have the distal opening of the working channel positioned behind the plane of the input face of the imaging assembly. See, e.g., U.S. Patent No. 9,775,675 to Irby, III ("Irby"), the disclosure of which is hereby incorporated by reference herein except for patent claims and express definitions contained therein. Irby teaches that, in order to decrease the distal head catheter shaft diameter, it is beneficial to terminate working channel behind the distal face. Conventional ureteroscopes typically define a viewing angle that is ±45 degrees from the axis of the catheter. Accordingly, conventional ureteroscopes do not include the inlet to the working channel within the viewing angle of the imaging assembly. This can compromise the functional visualization of the target zone.

Furthermore, successful laser ablation treatment of body stones requires contact or quasi contact between the laser fiber and the stone. For conventional laser ureteroscopes, such contact requires extending the distal tip of the laser fiber beyond the distal end of the catheter (typically 2 to 6 millimeters) in order for the operator to see and control exact position of the laser fiber with respect to stone surface during lithotripsy. The stone surface (and, preferably, the tip of the fiber) must be within the viewing angle of the imaging optics and also at the working distance of the imaging optics. Another important reason for extending and visualizing the fiber is prevention of soft tissue (mucosal) damage due to accidental ablation of soft tissue. Such ablation and perforation of the ureter or kidney may lead to a need for an open surgical intervention. A clear image of the distal tip of laser fiber and soft tissue surface can prevent soft tissue ablation accidents.

Various embodiments of the disclosure are configured so that the mouth of the working channel is within the viewing angle of the visualization system. In some embodiments, the use of a transparent cap provides a line of sight between the imaging receiver and the distal end of the laser fiber, enhancing the view of the operational area. The presence of the transparent cap also enables the line of sight to be unobscured by debris that is generated during the ablation process.

Conventional methods of laser lithotripsy include delivering laser radiation through a laser fiber to ablate the stone into very small particles ("dust") or fragments. The ablation can be performed in a contact or quasi-contact mode or in a non-contact ("popcorning") mode. The non-contact technique is typically used in conventional ureteroscopy for treatment of mid-sized and small stone fragments (typically below 3-5 millimeters in size) if retropulsion does not allow effective operation in the contact or quasi-contact mode. For the non-contact technique, the distal end of laser fiber is positioned at a fixed target zone close to the stone or fragments and the laser is activated without contact between the laser fiber and the stones or fragments. Vaporization and bubble implosions, as well as irrigation of the target zone, causes streaming of the liquid medium (primarily water) within the target zone, which in turn causes the smaller stone fragments to churn. The non-contact technique relies on the fragments or stones to enter the effective range of the laser emission within the fixed target zone for further ablative fragmentation and dusting.

Consider the limitations and effect of this conventional approach. Laser power is limited to relatively low levels in order to prevent overheating of the target zone and strong retropulsion effects. In the contact mode, retropulsion effects, especially for mid-sized stones or fragments, requires additional non-lasing time to trace or "chase" the targets, further lengthening the total time of treatment. It is difficult and time consuming to trace each and every one of such fragments. Non-contact mode is inefficient because actual ablation occurs only when the churning stones or fragments happen to be within an effective laser pulsing range of the distal tip of the fiber. Such "effective ablation" time interval typically constitutes only 10 - 30% of the total lasing time in non-contact mode. Stone free outcomes, which is the clinical goal of the treatment, is difficult to guarantee because some small fragments move out of the treatment zone due to the churning. Such limitations and effect of conventional laser lithotripsy prolong the total time of treatment and introduce safety risks due to the danger of overheating the liquid medium in the target zone.

Various embodiments of the disclosure enable shorter treatment times for laser lithotripsy because body stones are drawn to the laser fiber and there is less need to "chase" the body stone within the treated organ. The efficiency of breaking body stones is improved because of the drawing (suctioning) of stones and fragments towards the mouth of the aspiration channel and the distal end of an ablation laser fiber. The size, shape, and/or position of the irrigation outlets relative to the mouth may be configured to provide a flow field that enhances the entrainment of particles in the flow field that draws the body stones as well as the products of ablation into the mouth of the aspiration channel. Furthermore, in some embodiments, the irrigation flow may be adjusted in relation to the aspiration flow to provide such flow field continuously during the ablation treatment. To enhance monitoring of the ablation, the mouth of the aspiration channel may be positioned distal to an imaging receiver of the visualization system.

Also, collateral heat created by the process of laser ablation may be efficiently dissipated by the irrigation fluid and removed by the aspiration of the heated irrigation fluid, thereby reducing the risk of accidental thermal damage to surrounding tissues. The efficient dissipation of heat from the treatment zone further enables increased laser power without attendant increase in the risk of thermal injury to surrounding soft tissues.

Conventional flexible and semi-rigid endoscopes also include metal pull wires for imparting a bending angle at the distal end of the endoscope. The wires are attached to the distal end and are routed through the catheter to a steering mechanism. The wires have a footprint that occupies a portion of the cross-section of the catheter. Furthermore, the firm connection to the distal end requires connectors that also take up cross-sectional space at the distal end of the catheter. Also, steered catheters often require a torsion sleeve so that rotation of the shaft at the proximal end of the catheter translates to rotation of the distal end. The torsion sleeve also occupies a cross-sectional footprint. Such aspects of the steering and pointing system requires an increase in the total cross-section of the catheter, particularly at the distal end. Typical diameters of conventional ureteroscope are in the range of 3 to 4 millimeters. Further decreasing the diameter to the range of 1.7 to 2.5 millimeters may be accomplished by eliminating some functional elements, for example, steering components, such as disclosed by Irby.

Various embodiments of the disclosure present a distal head having a more compact radial profile than conventional endoscopes by eliminating need for pull wires and torsion sleeves. The use of illumination fibers for steering opens up cross-sectional space in the scope and specifically in the tip portion to allow use of both irrigation and aspiration channels within a common catheter shaft. In some embodiments, an illumination fiber is utilized not only for "pulling" on distal portions of the catheter, but also for "pushing" on the distal portions, thereby providing bidirectional steering with a single illumination fiber. This enables all the functions of the catheter-illumination, imaging, irrigation, aspiration, and ablation-within a cross-sectional dimension that is in a range of 2 to 2.5 millimeters inclusive. Cross-sectional dimensions in this range can enable ureteroscopic removal of body stones without subjecting the patient to a general anesthesia, as discussed by Irby.

Structurally, for various embodiments of the disclosure, a endoscopic surgical instrument is disclosed, comprising a catheter shaft defining and extending along a central axis and including a proximal portion and a distal portion, a distal head portion disposed at the distal portion of the catheter shaft, the distal head portion including a distal face, and a working channel extending within the catheter shaft from the proximal portion through the distal head portion, the distal head portion defining a mouth at the distal face, the working channel being configured to receive a laser fiber. An illuminator may be disposed at the distal head portion, and an imaging receiver disposed at the distal head portion, the imaging receiver being positioned at an axial distance proximal to a distal extremity of the distal face, the axial distance being within a range of 1 millimeter to 10 millimeters inclusive. In some embodiments, the mouth is at least partially within a viewing angle of the imaging receiver.

In some embodiments, the working channel is defined by and unitary with the catheter shaft. A laser fiber may be included for insertion into the working channel. In some embodiments, the catheter shaft includes a shaft cross-section normal to a central axis of the catheter shaft that defines an oblong shape, the shaft cross-section defining a major axis that passes through a maximum dimension of the oblong shape and a minor axis that is perpendicular to the major axis. In some embodiments, the maximum dimension of the shaft cross-section is in a range of 2.2 millimeters to 2.5 millimeters inclusive. In some embodiments, the minimum dimension of the shaft cross-section is in a range of 1.7 millimeters to 2.0 millimeters inclusive. The oblong shape may be an oval.

The distal head portion may includes a distal tip portion in contact with distal portion of the catheter shaft, the imaging receiver being mounted to the distal tip. In some embodiments, the distal tip portion includes the distal face. The distal tip portion may be unitary with the catheter shaft. In some embodiments, the distal head portion includes a transparent medium distal to and affixed to the distal tip portion, the transparent medium including the distal face. The mouth may at least partially visible through the transparent medium via the imaging receiver. In some embodiments, the working channel is an aspiration channel.

In some embodiments of the disclosure, an irrigation channel in fluid communication with an outlet, the outlet being defined by the distal head. The irrigation channel may be defined by an internal hollow of the catheter shaft exclusive of the aspiration channel, the internal hollow extending from the proximal portion of the catheter shaft to the distal portion of the catheter shaft. In some embodiments, the outlet of the irrigation channel is configured at an outlet angle relative to a distal direction along the central axis. The distal head portion includes a distal tip portion in contact with the distal portion of the catheter shaft, the outlet being defined by the distal tip portion. In some embodiments, the outlet angle is in a range of 0 degrees to 170 degrees inclusive; in some embodiments, the outlet angle is in a range of 10 degrees to 70 degrees inclusive; in some embodiments, the outlet angle is in a range of 20 degrees to 45 degrees inclusive.

The distal head portion may include a distal tip portion in contact with the distal portion of the catheter shaft and a transparent medium distal to and affixed to the distal tip portion, the outlet being defined by the distal tip portion and configured to direct irrigation flow onto a proximal face of the transparent medium. In some embodiments, a distal end of the laser fiber is selectively positionable over a range of axial positions relative to a distal-most location of the mouth. In some embodiments, the range of axial positions is not greater than 1 millimeter distal to the distal-most location of the mouth and not greater than 3 millimeters proximal to the distal-most location; in some embodiments, the range of axial positions is from flush with the distal-most location of the mouth and to not greater than 1 millimeter proximal to the distal extremity; in some embodiments, the range of axial positions is not less than 0.1 millimeter distal to the distal-most location of the distal tip and not greater than 0.6 millimeters proximal to the distal extremity. In some embodiments, the illuminator is a fiber optic, the fiber optic being anchored to the distal head portion. The catheter shaft may be flexible with the proximal portion of the catheter shaft coupled to a handle, the handle including a steering mechanism that is coupled to the distal head portion via the fiber optic for manipulation of the distal head portion.

In various embodiments of the disclosure, a surgical instrument is disclosed, comprising a catheter including a flexible catheter shaft coupled to a distal head, a first optical fiber extending through the catheter and into the distal head, the first optical fiber being anchored to the distal head, and a steering handle coupled to the catheter and the optical fiber, the steering handle being configured to exert forces on the first optical fiber for articulation of the distal head. The first optical fiber may be anchored to the distal head with an adhesive. In some embodiments, the first optical fiber defines an oblong cross-section defining a major dimension and a minor dimension, the major dimension being a maximum dimension of the oblong cross-section, the minor dimension being less than the major dimension and perpendicular to the major dimension at a central axis of the catheter.

In some embodiments, the surgical instrument includes a second optical fiber extending through the catheter and into the distal head, the second optical fiber being anchored to the distal head. The first optical fiber and the second optical fiber may be anchored within the distal head at locations that proximate an outer radial dimension of the distal head and are diametrically opposed about the central axis of the catheter and proximate an outer radial surface of the distal head. In some embodiments, the first optical fiber is one in a first bundle of optical fibers and the second optical fiber is one in a second bundle of optical fibers. Each of the first bundle of optical fibers and the second bundle of optical fibers may be arranged sequentially in a tangential direction about the central axis of the catheter at the distal head. Each of the first bundle of optical fibers and the second bundle of optical fibers may be centered about a respective plane at the distal head. In some embodiments, the first optical fiber and the second optical fiber each define an oblong cross-section defining a major dimension and a minor dimension, the major dimension being a maximum dimension of the oblong cross-section, the minor dimension being less than the major dimension and perpendicular to the major dimension at a central axis of the catheter. The major dimension may be in a range of 0.2 to 2.0 millimeters inclusive; the minor diameter may be in a range of 0.1 to 1.0 millimeters inclusive. In some embodiments, a ratio of the major diameter to the minor diameter is in a range of 2:1 and 5:1 inclusive.

In some embodiments of the disclosure, the steering handle includes a rotating cam directly coupled to the first optical fiber and the second optical fiber. In some embodiments, the first optical fiber is pulled in tension when the rotating cam is actuated in a first rotational direction to articulate the distal head in a first lateral direction, and the second optical fiber is pulled in tension when the rotating cam is actuated in a second rotational direction to articulate the distal head in a second lateral direction. The second rotational direction may be opposite the first rotational direction. Also, the second lateral direction may be opposite the first lateral direction. In some embodiments, the first optical fiber and the second optical fiber are bonded to the rotating cam. The rotating cam is coupled to a rotatable shaft and may be coupled to a thumb lever.

The first optical fiber and the second optical fiber may be operatively coupled to an illumination source and are routed from the illumination source to the rotating cam, and from the rotating cam to the distal head. In some embodiments, the illumination source is a light emitting diode. The illumination source may be housed within the steering handle. In some embodiments, the transparent medium defines a pressure relief that extends from the mouth. The pressure relief may extend radially to an outer perimeter of the transparent medium, and may extend radially to an outer perimeter of the distal face. In some embodiments, a pressure sensor is operatively coupled to the working channel. The optical fiber is configured to deliver visible light to a target zone that is distal to the distal head.

In various embodiments of the disclosure, an endoscopic surgical instrument for removing body stones from an internal organ is disclosed, comprising a catheter shaft that defines and extends along a central axis and having a proximal portion coupled to a handle, a distal tip portion coupled to a distal portion of the catheter shaft, a transparent medium coupled to the distal tip portion and including a distal face, and a working channel extending through the catheter shaft and the transparent medium from the proximal portion of the catheter shaft through the distal face of the transparent medium, the working channel defining a mouth. An illuminator may be disposed at the distal tip, and an imaging receiver disposed at the distal tip and proximal to the transparent medium. The distal face of the transparent medium may include a distal end of the working channel and is positioned from the imaging receiver at an axial distance that is in a range of 1 millimeter to 10 millimeters inclusive. In some embodiments, the distal end of the working channel is positioned from the imaging receiver at an axial distance that is in a range of 1.2 millimeters to 5 millimeters inclusive.

In some embodiments of the disclosure, an irrigation channel defines at least one outlet at the distal tip for directing irrigation flow at an angle relative to the central axis that is within a range of 0 degrees to170 degrees inclusive; in some embodiments the angle is within a range 10 degrees to- 70 degrees inclusive; in some embodiments, the angle is within a range 20 degrees to- 45 degrees inclusive.

Some embodiments include a laser fiber, a portion of which extends through the catheter shaft. The laser fiber may be inserted into the working channel. In some embodiments, the laser fiber is permanently integrated within the catheter shaft. A distal end of the laser fiber may be selectively positionable at axial positions ranging from 1 millimeter distal to a distal-most location of the mouth to 3 millimeters proximal to the distal face inclusive. In some embodiments, the axial positions range from flush with the distal face to 1 millimeter proximal to the distal face inclusive; in some embodiments, the axial positions range from 0.1 millimeter to 0.6 millimeter inclusive proximal to the distal face. A cross-sectional area of distal end of working channel may be in a range of 5% to 50% smaller than the cross-sectional area of working channel in the remaining part of the catheter shaft.

In some embodiments, the transparent medium defines a pressure relief that extends from the mouth. The pressure relief may extend radially to an outer perimeter of the transparent medium. In some embodiments, the pressure relief extends radially to an outer perimeter of the distal face. A pressure sensor may be operatively coupled to the working channel. In some embodiments, the working channel is defined by and unitary with the catheter shaft.

In various embodiments of the disclosure, a method for removing body stone material from an internal organ is disclosed, comprising: positioning a distal tip of a catheter assembly proximate a body stone material contained within an internal organ, the distal tip including a distal face that defines a mouth of a working channel of the catheter assembly, the body stone material being distal to the mouth; and positioning an imaging receiver proximal to the distal tip at a separation distance between the mouth and the imaging receiver while the distal tip is proximate the body stone material, the separation distance being in a range of 1 millimeter to 10 millimeters inclusive. In some embodiments, separation distance during the step of positioning an imaging receiver is in a range of 1.2 millimeters to 5 millimeters. Some embodiments include illuminating a target zone that surrounds the stone material with visible light. Some embodiments include obtaining an image of a targeted stone and the target zone using the imaging receiver. Some embodiments include positioning a laser fiber within the working channel, a distal end of the laser fiber being proximate the mouth. Some embodiments include selectively locating the distal end of the laser fiber within a range of distance that is not greater than 3 millimeters proximal to a distal-most location of the mouth and not greater than 1 millimeter distal to the distal-most location of the mouth, the range of distance being parallel to the axis of the working channel at the mouth; some embodiments include selectively locating the distal end of the laser fiber within a range of distance that is flush with the mouth and not greater than 1 millimeter proximal to the mouth, the range of distance being parallel to the axis of the working channel at the mouth.

Some embodiments include selectively locating the distal end of the laser fiber within a range of distance that is not greater than 0.6 millimeters proximal to the mouth and not less than 0.1 millimeter proximal to the mouth, the range of distance being parallel to the axis of the working channel at the mouth. Some embodiments include ablating the body stone material using the laser fiber. An average laser power delivered with the laser fiber during the method may be in a range of 120 Watts to 200 Watts inclusive. Some embodiments include operating the working channel as an aspiration channel, and removing products of ablation through the working channel. Some embodiments include delivering an irrigation fluid through the distal tip of the catheter. Some embodiments of the disclosure include delivering a flow of the irrigation fluid at a directed angle that is within a range of 0 degrees to 170 degrees inclusive relative to a distal direction along a central axis of the distal tip; some embodiments include delivering a flow of the irrigation fluid at a directed angle that is within a range of 10 degrees to 70 degrees inclusive relative to a distal direction along a central axis of the distal tip; some embodiments include delivering a flow of the irrigation fluid at a directed angle that is within a range of 20 degrees to 45 degrees relative to a distal direction along a central axis of the distal tip. During the method, the working channel may be an aspiration channel.

In various embodiments of the disclosure, a method for removing body stone material from an internal organ is disclosed, comprising providing a catheter assembly and providing operating instructions for the catheter assembly on a non-transitory, tangible medium, the operating instructions including: positioning a distal tip of a catheter assembly proximate a body stone material contained within an internal organ, the distal tip including a distal face that defines a mouth of a working channel of the catheter assembly, the body stone material being distal to the mouth; and positioning an imaging receiver proximal to the distal tip, wherein a separation distance between the mouth and the imaging receiver while the distal tip is proximate the body stone material is in a range of 1 millimeter to 10 millimeters inclusive. The operating instructions may include illuminating a target zone that surrounds the stone material with visible light, may include obtaining an image of a targeted stone and the target zone using the imaging receiver, and may include positioning a laser fiber within the working channel so that a distal end of the laser fiber is proximate the mouth. In some embodiments, the operating instructions include selectively locating the distal end of the laser fiber within a range of distance that is not greater than 3 millimeters proximal to a distal-most location of the mouth and not greater than 1 millimeter distal to the distal-most location of the mouth, the range of distance being parallel to the axis of the working channel at the mouth; in some embodiments, the operating instructions include selectively locating the distal end of the laser fiber within a range of distance that is flush with the mouth and not greater than 1 millimeter proximal to the mouth, the range of distance being parallel to the axis of the working channel at the mouth; in some embodiments, the operating instructions include selectively locating the distal end of the laser fiber within a range of distance that is not greater than 0.6 millimeters proximal to the mouth and not less than 0.1 millimeter proximal to the mouth, the range of distance being parallel to the axis of the working channel at the mouth. The operating instructions may include ablating the body stone material using the laser fiber, and may include delivering an average laser power in a range of 120 Watts to 200 Watts inclusive. In some embodiments, the operating instructions include removing products of ablation through the working channel, and may include delivering an irrigation fluid through the distal tip of the catheter. In some embodiments, the operating instructions include operating the catheter assembly to deliver a flow of the irrigation fluid at a directed angle that is within a range of 0 degrees to 170 degrees inclusive relative to a distal direction along a central axis of the distal tip; in some embodiments, the operating instructions include operating the catheter assembly to deliver a flow of the irrigation fluid at a directed angle that is within a range of 10 degrees to 70 degrees inclusive relative to a distal direction along a central axis of the distal tip; in some embodiments, the operating instructions include operating the catheter assembly to deliver a flow of the irrigation fluid at a directed angle that is within a range of 20 degrees to 45 degrees relative to a distal direction along a central axis of the distal tip. In some embodiments, the operating instructions include operating the working channel is an aspiration channel.

Various embodiments of the disclosure include a method of for removing body stone material from an internal organ, comprising: inserting an endoscopic surgical instrument that includes a catheter shaft defining and extending along a central axis, the catheter shaft including a proximal portion coupled to a handle and a distal tip portion at distal portion , the catheter shaft including an aspiration channel extending from the proximal portion to the distal tip portion with an imaging receiver disposed at the distal tip, the imaging receiver being positioned at an axial position that is in a range from 1 millimeter to 10 millimeters inclusive from a distal face of the distal tip portion, at least one illuminator disposed at the distal tip, a laser fiber disposed in the aspiration channel with a distal end of the laser fiber being extendable to a distance that ranges from 1 millimeter distal to the distal face of the distal tip to 3 millimeters proximal to the distal face, and an irrigation channel defined by an internal void that extends along a length of the catheter shaft, the irrigation channel and having an outlet at the distal tip that is configured to direct irrigation flow at an angle relative to the central axis that is in a range of 0 degrees to 170 degrees inclusive; obtaining an image of a targeted stone and surrounding area; placing the distal face proximate the body stone material; activating an irrigation flow through the irrigation channel; activating an aspiration flow through the aspiration channel to remove products of ablation through aspiration channel; and activating a laser coupled to the laser fiber to ablate targeted stone material.

### BRIEF DESCRIPTION OF THE DRAWINGS

FIG. 1 is a schematic of an endoscopic system for laser lithotripsy according to an embodiment of the disclosure;
FIG. 2 is an end view of a distal head portion for the endoscopic system of FIG. 1 that may be configured for a common irrigation and aspiration port according to an embodiment of the disclosure;
FIG. 2A is a sectional view of the distal head portion of FIG. 2 along plane IIA-IIA according to an embodiment of the disclosure;
FIG. 3 is an end view of a distal head portion for the endoscopic system of FIG. 1 that may be configured for separate irrigation and aspiration ports according to an embodiment of the disclosure;
FIG. 3A is a sectional view of the distal head portion of FIGS. 3, 4, and 5 along plane III-III according to an embodiment of the disclosure;
FIG. 3B is a sectional view of the distal head portion of FIGS. 3, 4, and 5 along plane III-III according to an embodiment of the disclosure;
FIG. 3C is a sectional view of the catheter along plane IIIC-IIIC of FIG. 3B according to an embodiment of the disclosure;
FIG. 4 is an end view of a distal head portion for the endoscope of FIG. 1 having illumination fiber optics that encroach on expanded irrigation ports of the distal head portion according to an embodiment of the disclosure;
FIG. 5 is an end view of a distal head portion for the endoscopic system of FIG. 1 having irrigation ports at an outer tangential perimeter of a transparent cap of a distal head portion according to an embodiment of the disclosure;
FIGS. 6 and 7 are end views of a distal head portions for the endoscopic system of FIG. 1 configured for irrigation ports coplanar with an aspiration port according to an embodiment of the disclosure;
FIG. 8 is a top view of a distal head portion with oblong irrigation ports at a distal tip of the catheter according to an embodiment of the disclosure;
FIG. 9 is a top view of a distal head portion for the endoscopic system of FIG. 1 with a reduced cross-section and oblong irrigation ports at a distal tip of the catheter according to an embodiment of the disclosure;
FIG. 10 is a perspective view of a distal head portion for the endoscopic system of FIG. 1 having a transparent cap and extension with pressure reliefs according to an embodiment of the disclosure;
FIG. 11is a side view of the distal head portion of FIG. 10 according to an embodiment of the disclosure;
FIG. 12 is a perspective view of a distal head portion for the endoscopic system of FIG. 1 having a transparent cap with irrigation ports and illumination fibers anchored thereto and integral pressure reliefs defined within the transparent cap according to an embodiment of the disclosure;
FIG.12A is a top view of the distal head portion of FIG. 12 according to an embodiment of the disclosure;
FIG. 12B is a side elevational view of the distal head portion of FIG. 12 according to an embodiment of the disclosure;
FIG. 13 is a top view of a distal head portion for the endoscopic system of FIG. 1 having a transparent cap with illumination fibers anchored thereto and integral pressure reliefs defined within the transparent cap according to an embodiment of the disclosure;
FIG. 14 is a side view of the distal head portion of FIG. 13 according to an embodiment of the disclosure;
FIG. 15 is a side view of the distal head portion of FIG. 13 depicting a flow field and diffusion of light from the illumination fiber optics according to an embodiment of the disclosure;
FIG.16 is an end view of a distal head portion for the endoscopic system of FIG. 1 having a single push-pull fiber optic configured to deflect the distal head portion for steering of the catheter according to an embodiment of the disclosure;
FIG. 16A is a sectional view of the distal head portion of FIG. 16 along plane XVIA-XVIA according to an embodiment of the disclosure;
FIG. 17 is a perspective view of a distal tip portion of the distal head portion of FIG. 16 in partial assembly with components extending through a catheter shaft, with an asymmetrically domed transparent cap depicted in phantom, according to an embodiment of the disclosure;
FIG. 18 is a cutaway view of the distal tip portion and catheter shaft of FIG. 17 according to an embodiment of the disclosure;
FIG. 19 is an elevational view of the components of FIG. 17 in assembly according to an embodiment of the disclosure;
FIG. 19A is an elevational view of an alternative to the assembly of FIG. 19 according to an embodiment of the disclosure;
FIG. 20 is an end view of a distal head portion for the endoscopic system of FIG. 1 without a transparent cap and having an image receiver axially offset from a mouth of the distal head portion according to an embodiment of the disclosure;
FIG. 20A is a sectional view of the distal head portion of FIG. 20 along plane XXA-XXA according to an embodiment of the disclosure;
FIG. 21 is an end view of a distal head portion for the endoscopic system of FIG. 1 without a transparent cap and having an image receiver axially offset from a mouth of the distal head portion and with dedicated irrigation ports according to an embodiment of the disclosure;
FIG. 21A is a sectional view of the distal head portion of FIG. 21 along plane XXIA-XXIA according to an embodiment of the disclosure;
FIG. 21B is a sectional view of the distal head portion of FIG. 21 along plane XXIB-XXIB according to an embodiment of the disclosure;
FIG. 21C is a sectional view of an alternative configuration for the distal head portion of FIG. 25 along plane XXIB-XXIB according to an embodiment of the disclosure;
FIGS. 22A through 22D are sectional views of lighting fiber optics having oblong cross-sections according to an embodiment of the disclosure;
FIG. 23 is a partial, internal view of a steering handle with push-pull fiber optic linkages mounted to a rotating cam and coupled to a light source according to an embodiment of the disclosure;
FIGS. 24A through 24C are schematic views of terminations for affixing fiber optic push linkages to the distal head portion according to embodiments of the disclosure;
FIG. 25A is a photograph of a target zone as viewed through the distal head portion of FIG. 10 with the transparent cap removed according to an embodiment of the disclosure;
FIGS. 25B is a photograph of a target zone as viewed through the distal head portion of FIG. 10 with a transparent cap having a cap thickness of 1 millimeter according to an embodiment of the disclosure;
FIGS. 25C is a photograph of a target zone as viewed through the distal head portion of FIG. 10 with a transparent cap having a cap thickness of 1.25 millimeter according to an embodiment of the disclosure; and
FIGS. 25D is a photograph of a target zone as viewed through the distal head portion of FIG. 10 with a transparent cap having a cap thickness of 1.5 millimeter according to an embodiment of the disclosure.

### DETAILED DESCRIPTION

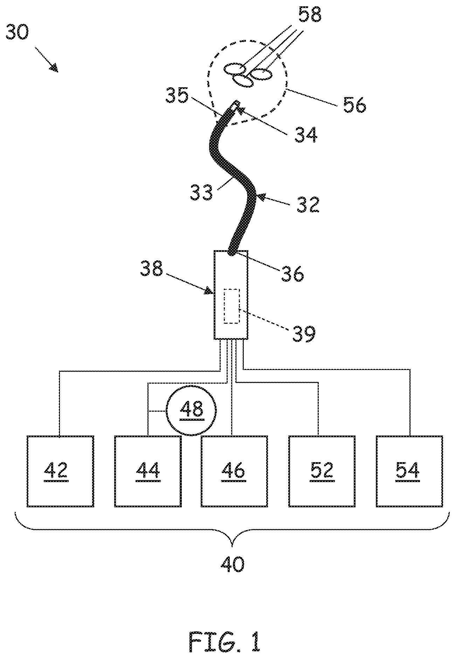

Referring to FIG. 1, an endoscopic system 30 for laser lithotripsy is schematically depicted according to an embodiment of the disclosure. The endoscopic system 30 includes a catheter 32 having a proximal portion 36 coupled to a handle 38 and a distal portion 35 that includes a distal head portion 34. The catheter 32 may include a catheter shaft 33 is that flexible (depicted), rigid, or semi-rigid. The handle 38 may house a steering mechanism 39 that is coupled to the distal head portion 34. The handle 38 integrates various external components or systems 40 for control and delivery to the distal head portion 34 via the catheter 32. The external systems 40 may include an irrigation system 42, a suction or aspiration system 44, an ablation laser system 46, an illumination system 52, and a visualization system 54. Some of the components of the endoscopic system 30 may be partially or completely integrated into the handle 38, the catheter 32, or the distal head portion 34. The handle 38, for example, may include control mechanism of the aspiration and irrigation systems 42 and 44, and a mechanism for adjusting position of the distal end of the laser fiber, as well as other components. The mechanism of fiber positioning may include a clamp (not depicted) that can be engaged once the distal tip of the fiber is in the desired position. Clamping the fiber fixes the position of the fiber distal tip, typically with an accuracy in the range of 0.05 to 0.1 millimeters. The direction from the catheter shaft 33 to the distal head portion 34 along a central axis 110 is herein referred to as the distal direction 50. The direction opposite the distal direction 50 is herein referred to as the proximal direction 51.

Functionally, the steering mechanism 39 enables articulation of the distal portion 35 of the catheter 32, particularly for embodiments incorporating a catheter shaft 33 that is flexible or semi-flexible, for routing through body vessels of the patient to a target zone 56 and for alignment of the distal head portion 34 to hone in on individual body stones 58 within the target zone 56. The illumination system 52 generates visible light that is delivered to the target zone 56 for illumination of the body stones 58 and surrounding tissue, for example stones within a kidney, ureter or bladder. The ablation laser system 46 includes, for example, a Thulium or Holmium fiber or solid state laser, for delivering laser energy to the target zone 56 for ablation and break up of body stones 58. Delivery of the laser energy may be accomplished using a laser fiber, for example, silica or other optical fiber material. The irrigation system 42 provides pressurized irrigation fluid for cooling of the target zone 56 and for moving fragments of body stones 58 within the target zone 56. The aspiration system 44 draws liquid medium away from the target zone 56, including particles from the body stones 58 that may be suspended in the medium. In some embodiments, the aspiration system 44 includes a pressure sensor 48 that monitors the aspiration pressure. Pressure sensors may also be utilized to monitor the irrigation pressure.

Herein, "body stones" encompass any stone that is produced by the human body, including kidney stones and ureteral stones, as well as species thereof including calcium stones, uric acid stones, struvite stones, and cysteine stones. "Body stones" may also include stones found in or formed by other organs of the body, for example, bladder stones, gallbladder stones, prostate stones, pancreas stones, saliva gland stones, and belly stones. The present disclosure describes, but in general is not limited to, systems and techniques for breakup of kidney and ureteral stones. In view of this disclosure, those of skill in body stone therapies will recognize the application of various aspects disclosed herein for the remediation of body stones other than kidney and ureteral stones as well as for treatment of hard and soft tissues.

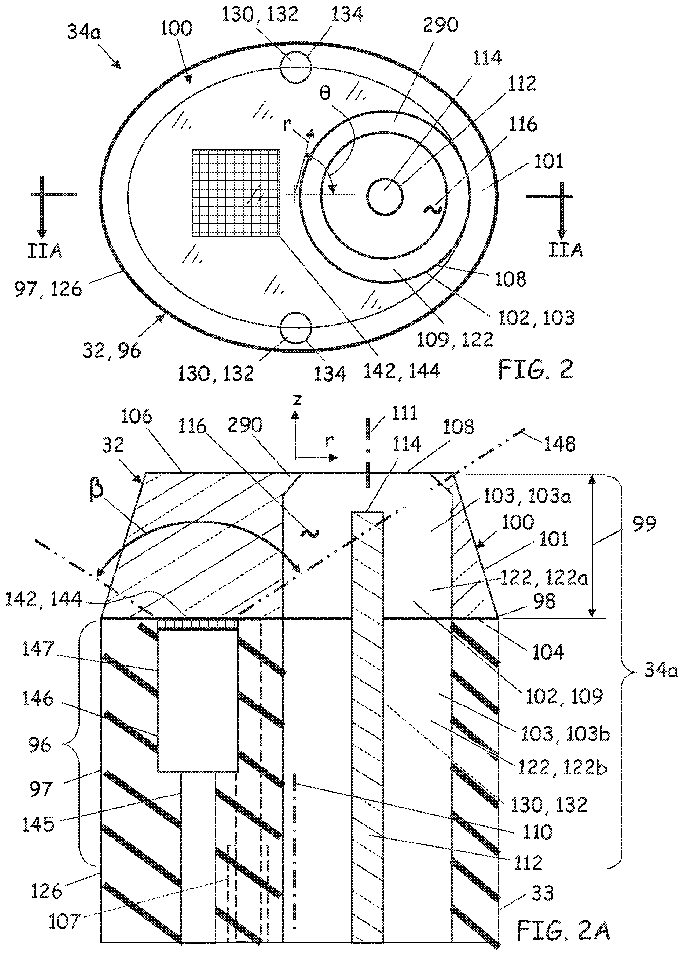

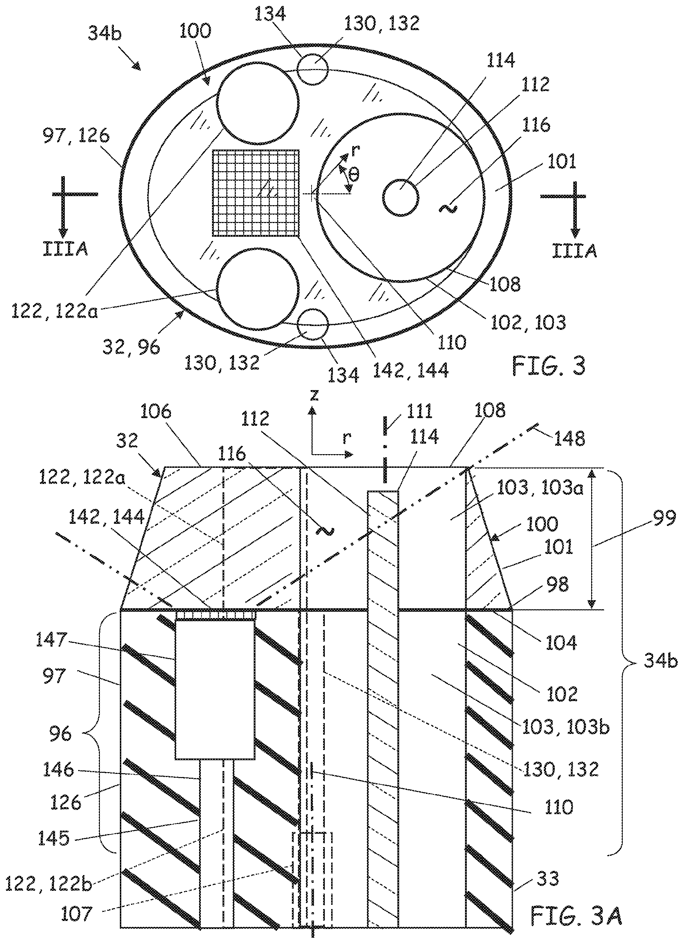

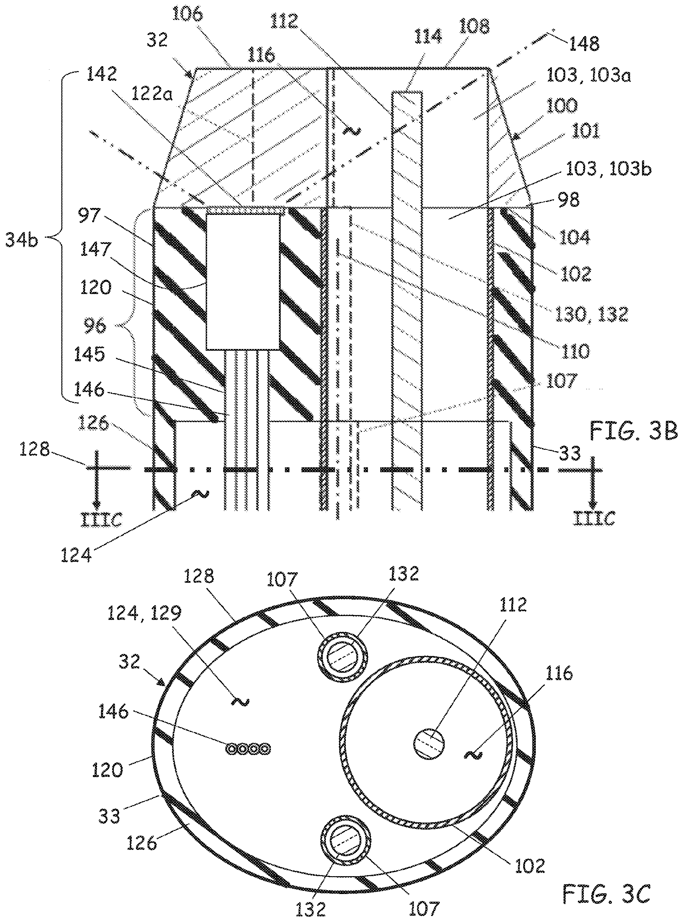

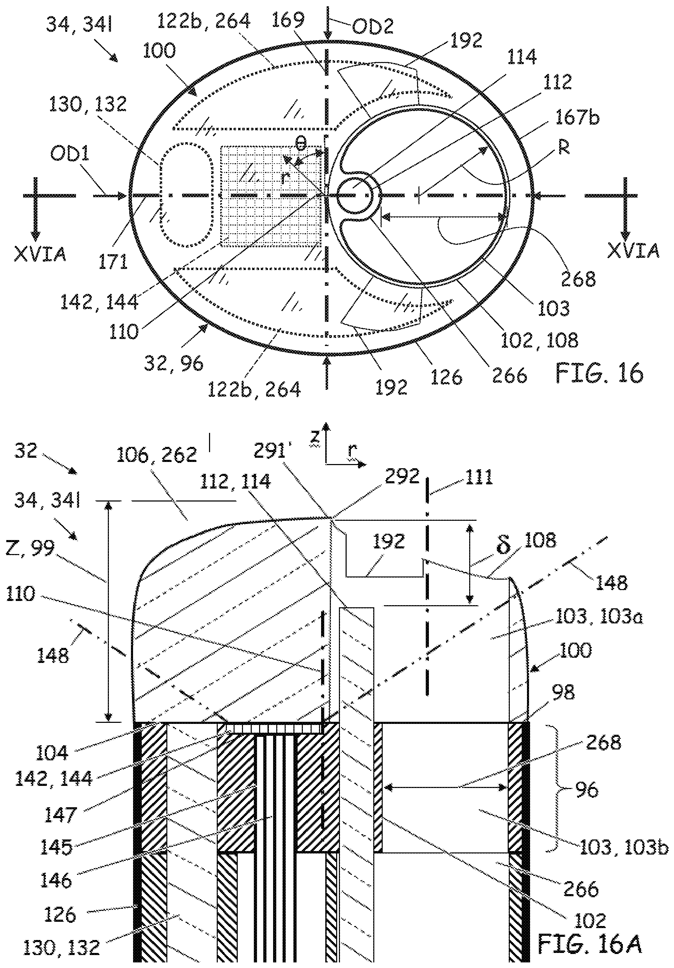

Referring to FIGS. 2 and 2A, a distal head portion 34a is depicted according to an embodiment of the disclosure. Herein, distal head portion(s) are referred to collectively or generically by reference character 34, whereas individual or specific embodiments of the distal head portion are referred by reference character 34 followed by a letter suffix (e.g., "distal head portion 34a"). The distal head portion 34a includes a distal tip portion 96 having a distal face 98 and an outer tangential surface 97. In some embodiments, the distal tip portion 96 is unitary with the catheter shaft 33 (e.g., FIGS. 2A, 3A, and 3B); in other embodiments, the distal tip portion 96 is formed separate from the catheter shaft 33 and affixed thereto (e.g., FIGS. 16 - 21C). In some embodiments, a transparent cap portion 100 is secured to the distal face 98 of the distal tip portion 96. The transparent cap portion 100 includes a proximal face 104 and a distal face 106 that defines an axial cap thickness 99 therebetween. In some embodiments, the transparent cap portion 100 defines an inclined surface 101 that extends proximally from the distal face 106, for example, a chamfer (depicted) or arcuate corner. The transparent cap portion 100 is fabricated from a material appropriate for transmitting visible light and may include a low absorptivity and high damage threshold at the operating wavelengths of the ablation laser system 46. Non-limiting example materials for the transparent cap 100 include sapphire, quartz, optical ceramic, and mineral or organic glass. In some embodiments, the refractive index of the transparent cap 100 is about 1.31 to 1.35, to approximately match the refractive index of the liquid medium (substantially water). In some embodiments, the distal tip 96 may be fabricated from the same transparent material as the transparent cap 100.

In some embodiments, the distal head portion 34a contains one or more illuminators 130. The illuminators 130 may be the distal end of an illumination or lighting fiber optic 132 for transmitting light in the visible spectrum and is operatively coupled to the illumination system 52 at the handle 38. The illumination fiber optic 132 pass through an illumination fiber optic port 134 formed in the distal tip portion 96 and may extend into the transparent cap 100. Optionally, the illuminator 130 may be light emitting diodes (LEDs) (not depicted) that are proximate the proximal face 104 of the transparent cap 100 and are sourced by an electrical lead that extends through the catheter 32. The illumination fiber optics 132 act as optical waveguides and may extend through the catheter 32 and be coupled to the illumination system 52 at the handle 38.

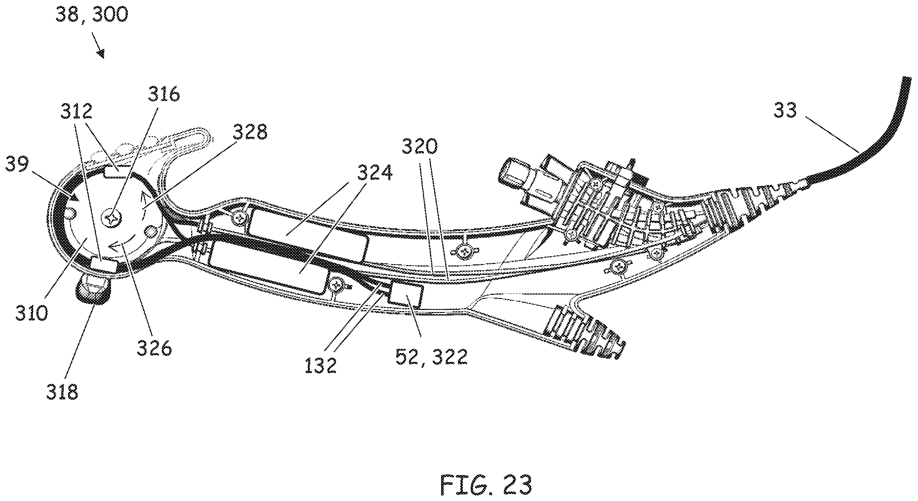

In some embodiments, one or more illumination fiber optics 132 are mechanically affixed to the distal head portion 34a (e.g., with an adhesive), for example, to the illumination fiber optic port 134 or the transparent cap 100 or both. The fiber optic(s) 132 may extend through and remain free to slide within lumens 107 (FIGS. 2A, 3A, and 3C) that are defined by or disposed within the catheter 32. The illumination fiber optics 132 may extend distally from the steering mechanism 39 disposed within the handle 38 for translation within the lumen 107. (An example of the steering mechanism 39 is described attendant to FIG. 23.) The distal head portion 34d is thereby coupled to the steering mechanism 39 of the handle 38 via the illumination fiber optic(s) 132. For catheters 32 with shafts 33 that are flexible or semi flexible, the coupling and routing of the illumination fiber optics 132 so arranged enables the illumination fiber optic(s) 132 to also serve as a pulling linkage or a push-pull linkage for steering of the distal head portion 34d, thereby negating the need for separate pull wires and the connectors associated with coupling them to the distal head portion 34a.

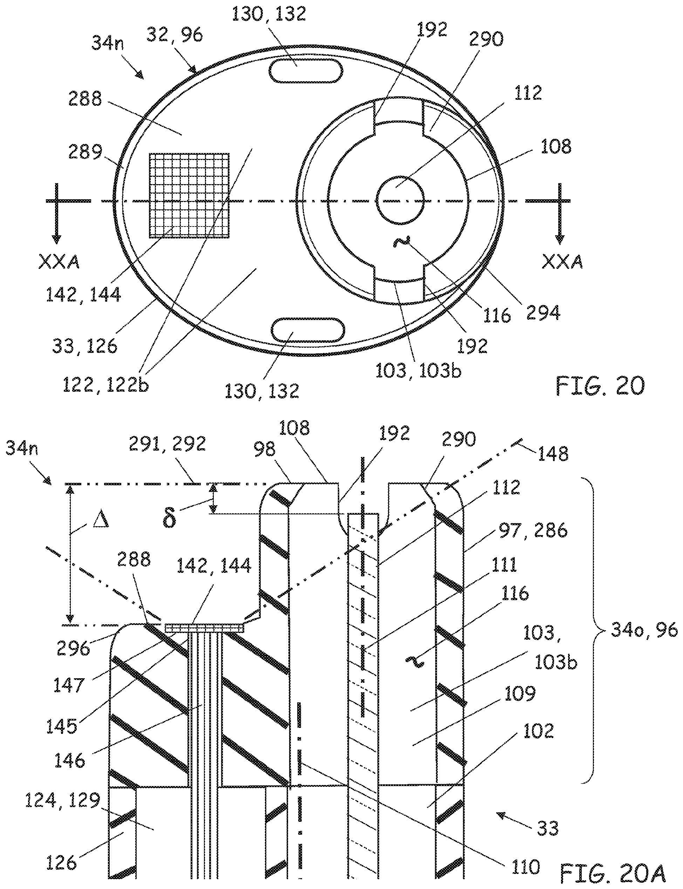

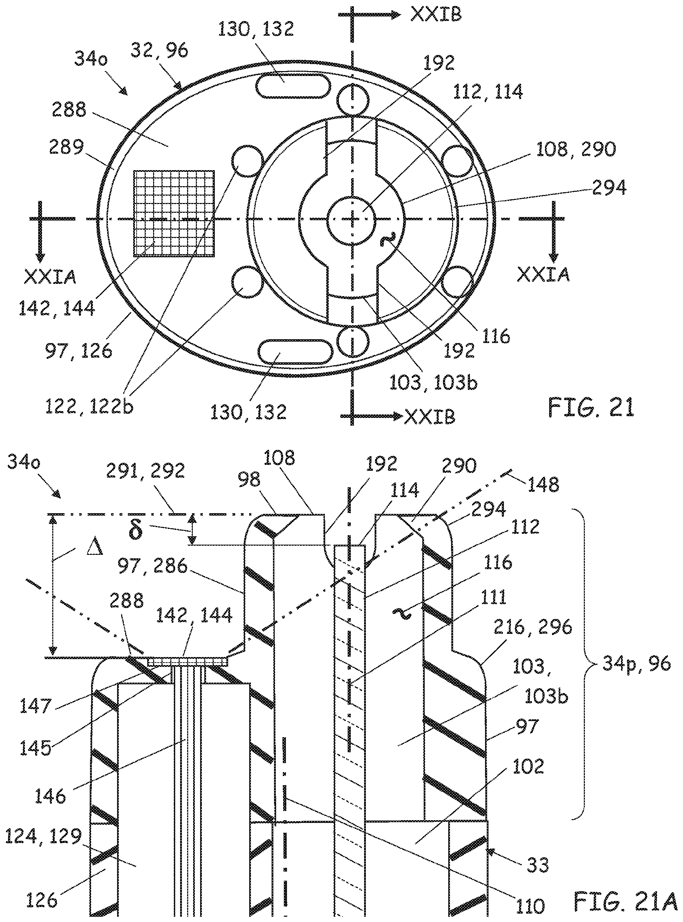

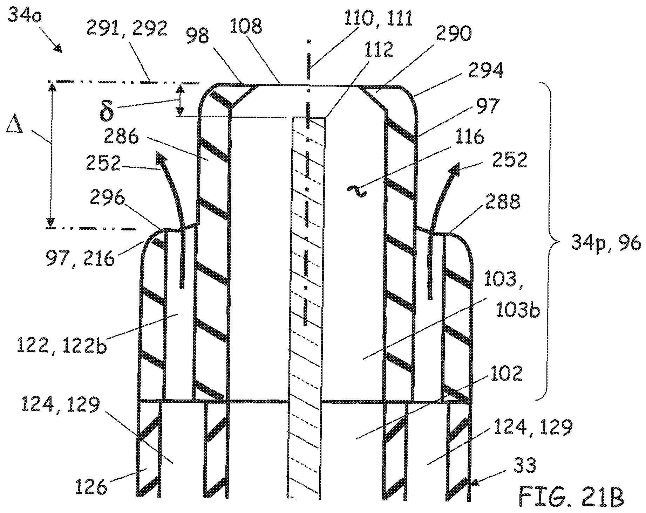

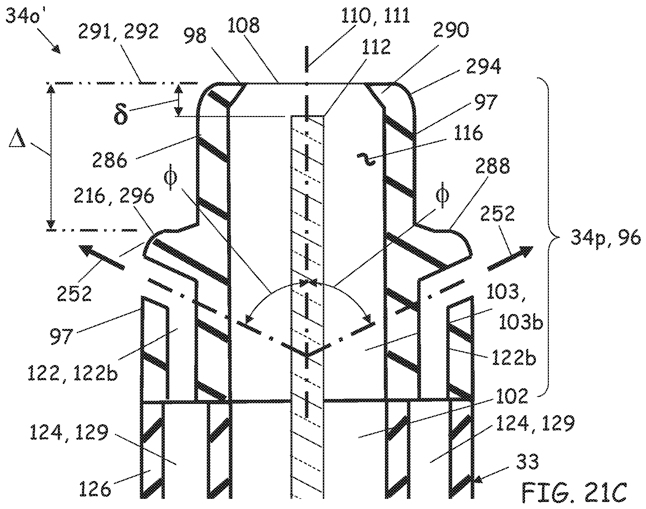

The distal head portion 34a defines a working channel 102 that passes through the distal tip portion 96 and through the proximal face 104 and the distal face 106 of the transparent cap portion 100. The working channel 102 defines a mouth 108 at the distal face 106. The working channel 102 may serve, for example, as an aspiration port, in which case the mouth 108 and working channel define an aspiration inlet. The working channel 102 extends through the catheter 32 and may be coupled, for example, to the aspiration system 44 at the handle 38. The distal head portion 34a may define, for example, a round or oblong cross-section that defines and is concentric about a central axis 110. The working channel 102 includes a working port 103 that is formed in and passes through the distal head portion 34a and defines the mouth 108. In some embodiments, the working port 103 includes a cap working port 103a and a distal tip working port 103b that are in fluid communication with each other. The cap working port 103a passes through the transparent cap 100, defining a cap working port axis 111. In some embodiments, the distal tip working port 103b passes through the distal tip portion 96 to transition between the catheter shaft 33 and the transparent cap 100. Alternatively, embodiments where the transparent cap 100 is coupled directly to the catheter shaft 33 are also contemplated (e.g., without a transitioning of the distal tip portion), such that the working port 103 comprises only the cap working port 103a. Embodiments where the distal head 34 includes a distal tip portion 96 without a transparent cap are also disclosed herein. (See FIGS. 20 and 21 below and attendant discussion.)

A laser fiber optic 112 for transmitting ablative laser energy is disposed in the working channel 102, a distal end 114 of the laser fiber optic 112 being positioned proximate the distal face 106 of the transparent cap portion 100, and a proximal end of the laser fiber optic 112 being coupled to the ablation laser system 46 via the handle 38. A core diameter of the laser fiber optic 112 may be in a range of 0.05 to 0.4 millimeters for a catheter having a flexible shaft and up to 1.5 millimeters a catheter having a rigid shaft. In some embodiments, the laser fiber optic 112 is substantially concentric with the cap working port axis 111 or otherwise extends through a center portion of cap working port 103a to define an annular region 116 between the laser fiber optic 112 and the cap working port 103a. In some embodiments, the position of the distal end 114 of the laser fiber optic 112 can be controlled within a range of +/- 5 millimeter inclusive relative to the distal face 106 of the transparent cap portion 100, where "+" and "-" refer respectively to the distal and proximal directions 50 and 51 along the working port axis 111. In some embodiments, the position of the distal end 114 can be controlled within a range of +/- 3 millimeter inclusive relative to the distal face 106. In some embodiments, the position of the distal end 114 be controlled within a range of +1 to - 3 millimeter inclusive relative to the distal face 106. In some embodiments, the position of the distal end 114 can be controlled within a range of -0 to - 3 millimeter inclusive relative to the distal face 106. In some embodiments, the position of the distal end 114 can be controlled within a range of -0.05 to - 1 millimeter inclusive relative to the distal face 106. Herein, a range that is said to be "inclusive" includes the endpoint values of the range as well as all values between the endpoint values.

In some embodiments, one or more working ports 122 are defined that extend through the transparent distal head portion 34. The working port 103 and the working port 122 may be plumbed to a common working channel 109, as depicted in FIGS. 2 and 2A. In some embodiments, the working channel 109 serves alternately as an aspiration and an irrigation channel. Herein, a "working channel" may serve as an irrigation channel, an aspiration channel, or both. Working channels as used herein may optionally be configured to accommodate working objects such as laser fibers and baskets. The inner diameter of the working port 103 may be in the range from 0.5 to 1.5 millimeters inclusive for flexible catheters utilizing a 0.05 millimeter core laser fiber.

Akin to the working port 103, each of the working ports 122 may comprise a cap working port 122a and a distal tip working port 122b that are in fluid communication with each other. The cap working port(s) 122a passes through the transparent cap 100. In some embodiments, the distal tip working port(s) 122b passes through the distal tip portion 96 to transition between the catheter shaft 33 and the transparent cap 100. Alternatively, embodiments where the transparent cap 100 is coupled directly to the catheter shaft 33 are also contemplated (e.g., without a transitioning of the distal tip portion), such that the working port(s) 122 comprises only the cap working port(s) 122a.

In some embodiments, the distal head portion 34a includes an imaging receiver 142, which may include image-forming optics defining a field of view 148 of the endoscopic system 30, characterized by a viewing angle β. In some embodiments, the imaging receiver 142 defines a viewing angle β that is within a range of 90 to 120 degrees inclusive (±45 to 60 degrees inclusive from the viewing axis of the imaging receiver). The imaging receiver 142 may be an imaging device 144 (depicted), such as a complementary metal oxide semiconductor (CMOS) sensor (including a semiconductor chip, imaging optics, and supporting electronics) or a charge-coupled device (CCD) camera sensor. In some embodiments, the imaging face the imaging receiver 142 is from 0.5 x 0.5 millimeter to 1.5. x 1.5 millimeter. An example of the described CMOS image sensor is the NANEYE 2D supplied by AWAIBA CMOS Image Sensors of Argau, Switzerland. See https://ams.com/naneye, last visited January 16, 2020.

The imaging device 144 may include a cable 146 that extends through the catheter 32 and may be coupled to the visualization system 54 at the handle 38. The cable 146 may be routed through a cable port 145 defined by the distal tip 96. In some embodiments, the imaging device 144 is disposed in a recess 147 at the distal face 98 of the distal tip portion 96. Imaging devices 144 may define a viewing angle β that is ±45 degrees of normal. Optionally, the imaging receiver 142 is a distal end of an optical system and imaging fiber optic (not depicted) which extends through the catheter 32 and is coupled to the visualization system 54 at the handle 38. The distal face 106 of the transparent cap 100 may be flat (depicted) or, alternatively, shaped as a lens (not depicted) for imaging onto the imaging receiver 142.

Referring to FIGS. 3 and 3A, a distal head portion 34b is depicted according to an embodiment of the disclosure. The distal head portion 34b may include many of the same components and attributes as the distal head portion 34a, which are indicated with same-numbered reference characters. A distinction of the distal head portion 34b is that the working ports 122 are separate from the working port 103. In some embodiments, an inner diameter of the working port 122 for irrigation is in a range of 0.5 to 1.5 millimeters inclusive. Functionally, having separate ports 103 and 122 serviced by separate working channels 102 and 124 enables irrigation and aspiration to occur simultaneously and continuously during laser treatment.

Referring to FIGS. 3B and 3C, the distal head portion 34b is depicted with a distal tip portion 96 and catheter 32 having a tubular shaft 120 according to an embodiment of the disclosure. In the embodiment of FIGS. 3B and 3C, the working port(s) 122 are in fluid communication with a single working channel 124 that is bound by an outer portion 126 of the catheter shaft 33. That is, in some embodiments, the catheter shaft 33 defines a cross-section 128 normal to the central axis 110 defining a hollow 129 that extends from the proximal portion 36 to the distal portion 35, the hollow 129 being occupied by various components that service the distal head portion 34 of the catheter 32. The occupying components may include, but are not limited to, the working channel 102, the laser fiber optic 112, the illumination fiber optic(s) 132 and lumen(s) 107, and cable 146. Sterilization of the hollow 129 may be performed for single-use endoscopes by an ethylene oxide (ETO) gas sterilization process.

By this arrangement, the working channel 102 is disposed within and is effectively surrounded by the single working channel 124. The irrigation system 42 may be coupled to the catheter shaft 33 so that irrigation fluid can flow through the balance of the hollow 129 that is not occupied by the components. The tubular shaft 120 may be implemented with any of the distal head portions 34 depicted at FIGS. 3 through 9.

For the various disclosed endoscopic systems 30 that implement aspiration and irrigation simultaneously, the total treatment time can be reduced while the safety of the procedure is enhanced. A method according to an embodiment of the disclosure may include some or all of the following:
(1) Identifying a stone in the internal organ of the patient using ultrasound, fluoroscopy or other diagnostic methods available to the artisan;
(2) Inserting the catheter 32 into the body of the patient and bringing the distal end of the catheter into the proximity of the target zone 56;
(3) Obtaining an image of a targeted body stone 58 or stone fragment;
(4) Bringing the distal end 114 of the laser fiber optic 112 into contact or quasi contact with the targeted body stone or fragment;
(5) Activating an irrigation flow and an aspiration flow; and
(6) Delivering laser energy from the ablation laser system 46 through the laser fiber 112 to ablate stone 58 into the large fragments (greater than 1 millimeter), small fragments (less than 1 millimeter) or particles (less than 0.25 millimeter).
The method above may be used for contact as well as non-contact treatment of body stones 58.

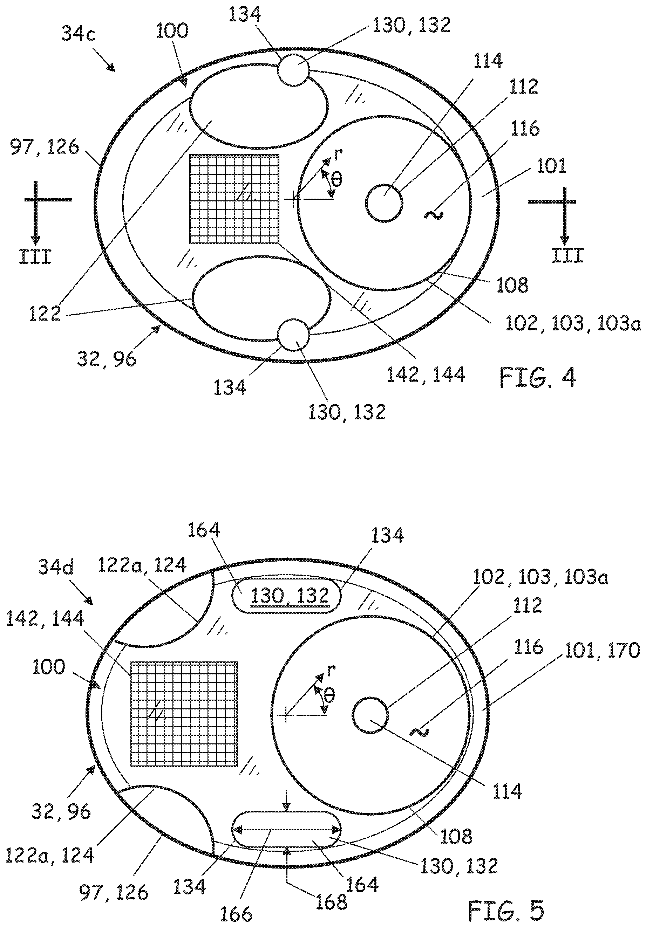

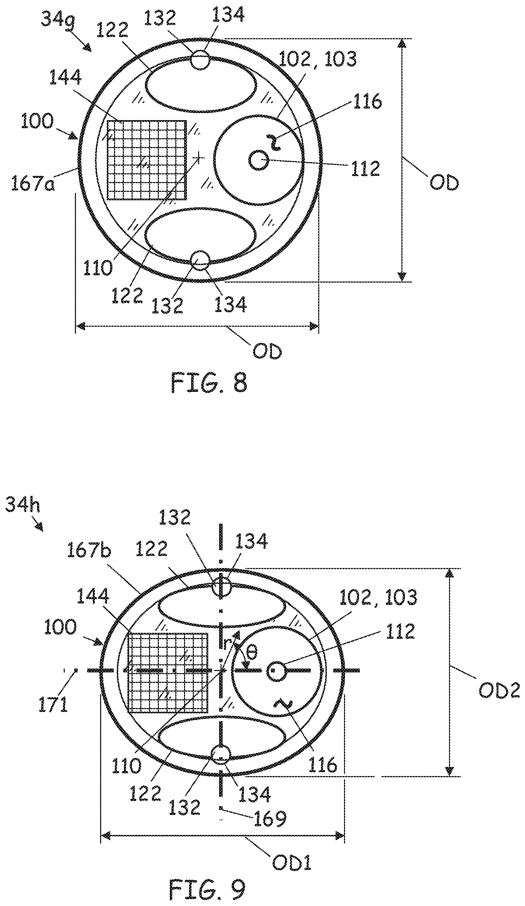

Referring to FIG. 4, distal head portion 34c is depicted according to embodiment of the disclosure. The distal head portion 34c may include many of the same components and attributes as the distal head portion 34b, which are indicated with same-numbered reference characters. A distinction of the distal head portion 34c is that the illumination fiber optic ports 134 and the working ports 122 overlap so that the illumination fiber optics 132 encroach on the boundary of the working ports 122. A further distinction of the distal head portion 34c is that the working ports 122 are shaped to increase the flow cross-section without increasing the overall profile of the distal head portion 34c. In the depicted embodiment, the working ports 122 of the distal head portion 34c are oblong to accomplish the increase, but other shapes are contemplated, including port cross-sections that are asymmetric. Additional discussion of the asymmetric working port 122 aspect is discussed below attendant to FIGS. 8 and 9.

Functionally, positioning the distal end 114 of the laser fiber 112 inside the distal head 34 protects the distal end 114 of the fiber from damage by stone ablation products, and can also increase the laser ablation efficiency while decreasing the total laser treatment time. Such placement minimizes or excludes fiber burn back and eliminates the need to reposition the fiber distal end 114 during the laser procedure. The transparent cap 100 provides a clear visual path between the imaging receiver 142 and the distal face 106 of the transparent cap 100, thus eliminating or substantially reducing the debris (e.g., ablation particles) within the near field of view 148 that would otherwise be present between the imaging receiver 142 and the laser fiber optic 112. The reduction of debris in the near field of view 148 enables the operator to better visualize the mouth 108, the distal end 114 of the laser fiber optic 112, and a given targeted body stone 58, and also reduces the attenuation of the light emitted by the illuminator(s) 130 for better illumination of the target zone 56. Also, the distal face 106 of the transparent cap 100, which can be more readily visualized than the smaller distal end 114 of the laser fiber optic 112, can assist the operator with positioning of the distal head portion 34a for better control of the distance between the distal end 114 of the laser fiber optic 112 and the targeted body stone 58. The improved control leads to increased ablation efficiency, as there is little or no gap between the distal end 114 and the targeted body stone 58 or fragment (said gap typically not exceeding 1 millimeter). The reduction of debris in the near field of view 148 also reduces the attenuation of the light from the illuminator(s) 130 for better illumination of the target zone 56 and a clearer view of the image of the target zone 56. Disposing the imaging device 144 in the recess 147 enables the proximal face 104 of the transparent cap to be planar to seat with the distal face 98 of the distal tip portion 96. The inclined surface 101 reduces the trauma of passing the distal head portion 34a through bodily vessels en route to the target zone 56.

Coupling to the steering mechanism 39 of the handle 38 via the illumination fiber optics 132 enables the illumination fiber optics 132 to also serve as the pull linkage and, in some embodiments, as a push-pull linkage for steering catheters 32 having shafts 33 that are flexible or semi-rigid. The need for separate pull wires and the connectors associated with coupling them to the distal head portion 34d is thereby negated, enabling more cross-section to be devoted to working channels, or reducing the cross-sectional profile of the catheter 32, or a combination thereof. Arranging the illumination fiber 132 so as to encroach on the boundary of the working ports 122 provides more cross-sectional area for irrigation flow.

By disposing the laser fiber optic 112 in the working channel 102, the distal end 114 can be recessed relative to the distal face 106 of the transparent cap 100 because the suction of the solution into the working channel 102 tends to draw the body stone 58 toward the laser fiber optic 112. Recessing the distal end 114 mechanically protects the laser fiber optic 112 during insertion and operation. In some embodiments, the distal end 114 of the laser fiber 112 can oscillate laterally during the laser treatment due to forces of irrigation or aspiration flow as well as laser-induced bubbling and streaming in the liquid. Such oscillations may be desirable and can be controlled through controlling parameters of the laser as well as the irrigation and/or aspiration flow (e.g., by modulating the flow rate).

Also, drawing the body stones 58 toward the laser fiber optic 112 can reduce or overcome "retropulsion" effects that develop when the heat of ablation forms vapor pockets on the ablated face of the body stone 58. Retropulsion effects are described in greater detail at International Application No. PCT/US19/42491 to Altshuler, et al., filed July 18, 2019 and owned by the owner of the present application, the disclosure of which is hereby incorporated by reference herein in its entirety except for express definitions and patent claims contained therein. Furthermore, because distal end 114 can be viewed through the transparent cap 100, visualization and control of the distance between the distal end 114 of the laser fiber optic 112 and the targeted body stone 58 is not compromised. In addition, collateral heat created by the process of laser ablation may be efficiently dissipated by the irrigation fluid and removed by the aspiration of the heated irrigation fluid through the working channel 102, thereby reducing the risk of accidental thermal damage to surrounding tissues.

Referring to FIG. 5, a distal head portion 34d is depicted according to an embodiment of the disclosure. The distal head portion 34d includes many of the same components and attributes as the distal head portion 34a, which are indicated by same-numbered reference characters. Like the distal head portion 34a, distal head portion 34d may utilize the illumination fiber optics 132 as push-pull elements for steering catheters 32 having shafts 33 that are flexible. In some embodiments, the illumination fiber optics 132 have an oblong cross section 164. Generally, an "oblong" cross section 164 has a major dimension 166 and a minor dimension 168 that are perpendicular to each other, the major dimension 166 being the greatest dimension of the oblong cross section 164 and the minor dimension 168 being a minimum dimension that is perpendicular to the major dimension 166 and being specified as less than the major dimension 166.

In some embodiments, the major dimension 166 of the oblong cross section 164 extends tangentially (i.e., substantially parallel to a tangential direction θ relative to the central axis 110 of the distal head portion 34d) and the minor dimension 168 extends radially (i.e., parallel to a radial direction r relative to the central axis 110 of the distal head portion 34d). In the depicted embodiment, working ports 122a may be disposed at an outer tangential perimeter 170 of the transparent cap 100, the working ports 122a passing through the proximal face 104 and the distal face 106 of the transparent cap 100 and being open at the distal face 106 and along the outer tangential perimeter 170 of the transparent cap 100 (e.g., along the inclined surface 101).

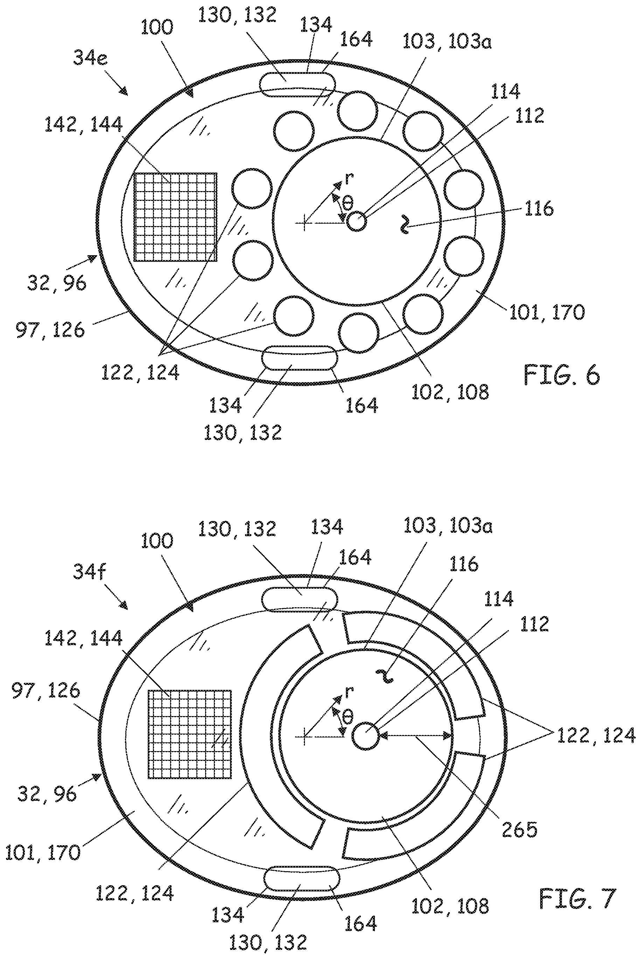

Referring to FIGS. 6 and 7, distal head portions 34e and 34f utilizing illumination fiber optics 132 having oblong cross-sections 164 and working ports 122 proximate the annular region 116 of the working port 103 are depicted according to an embodiment of the disclosure. Distal head portions 34e and 34f may include many of the same components and attributes as the distal head portion 34d, which are indicated with same-numbered reference characters. A distinction of the distal head portions 34e and 34f is that working ports 122 surround the annular region 116. Like the distal head portion 34a, distal head portions 34e and 34f may utilize the illumination fiber optics 132 as push-pull elements for steering catheters 32 having shafts 33 that are flexible. For the distal head portion 34e, the working ports 122 are circular. For the distal head portion 34f, the working ports 122 are arcuate. A plurality of the working ports 122, such as depicted at FIGS. 3 through 9, may be sourced by irrigation flow through the single working channel 124. In some embodiments, a ratio of the areas of the working ports 122 to the mouth 108 is within a range of 1.2 to 3.0 inclusive.

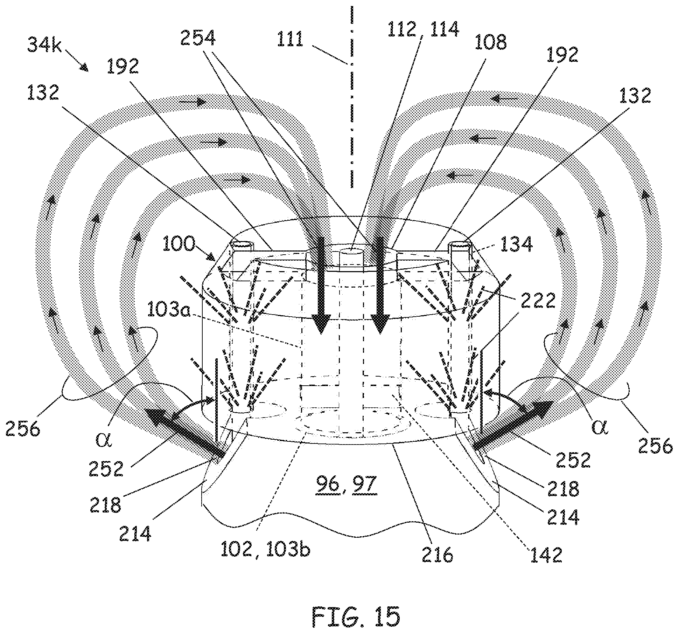

Functionally, when the working channel 102 is utilized for aspiration, the proximity of the working ports 122 surrounding the mouth 108 creates a flow field 256 that flows outward from the working ports 122 and folds inward toward the mouth 108. The flow field concept is discussed further attendant to FIG. 15.

Referring to FIGS. 8 and 9, distal head portions 34g and 34h are depicted to illustrate general aspects of the layout of the working ports 122 according to embodiments of the disclosure. The head portion 34g and distal tip portion 96 of the catheter 32 define a circular cross-section 167a (FIG. 8) that is normal to the central axis 110. The working ports 122 may be oblong to provide a larger flow cross-section than would be provided by circular irrigation ports. The circular distal head portion 34g is characterized by a substantially uniform outer dimension OD. The head portion 34h and distal tip portion 96 define an oblong cross-section 167b (FIG. 9 and elsewhere) such as an oval, elliptic, obround, or rounded rectangle cross-section.

The oblong cross-section 167b is achieved by locating the working ports 122 and illumination fiber optics 132 closer to the central axis 110, so that the oblong cross-section 167b has a reduced profile (i.e., has less cross-sectional area) relative to the circular cross-section 167a. The oblong cross-section 167b defines a major axis 171 that passes through a maximum outer dimension OD1 of the oblong cross-section 167b and a minor axis 169 that is perpendicular to the major axis 171. The minor axis 169 may define a minimum outer dimension OD2 of the oblong cross-section 167b. In some embodiments, the outer dimensions OD, OD1 of the cross-sections 167a, 167b are in a range of 2 to 3.2 millimeter inclusive; in some embodiments, the outer dimensions OD, OD1 are in a range of 1.7 millimeters to 2.6 inclusive; in some embodiments, the outer dimensions OD, OD1 are in a range of 2.2 to 2.5 millimeters inclusive. In some embodiments, the outer dimension OD2 of the cross-section 167b is in a range of 1.7 to 2.5 millimeters inclusive; in some embodiments, the outer dimension OD2 is in a range of 1.7 to 2.0 millimeters.

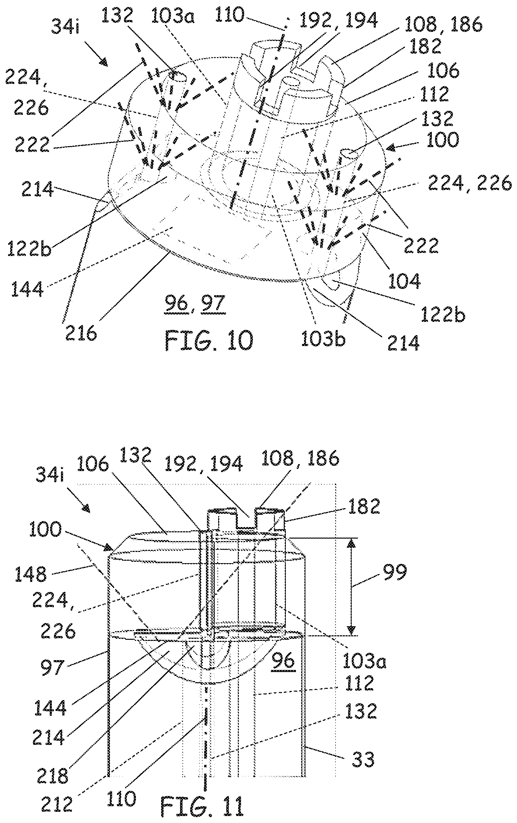

Referring to FIGS. 10 and 11, a distal head portion 34i having an extension 182 of the working port 103 is depicted according to an embodiment of the disclosure. The distal head portion 34i includes many of the same components and attributes as the distal head portion 34b, which are identified with same-numbered reference characters. The cap working port 103a defines the mouth 108 proximate the distal face 106 of the transparent cap 100. For the distal head portion 34i, the mouth 108 of the cap working port 103a is defined at a distal extremity 186 of the extension 182. At least one pressure relief 192 extends proximally from the mouth 108. The pressure relief(s) 192 may be a notch or notches 194. The notches may extend radially through a wall 196 of the extension 182.

For the distal head portion 34i, the distal tip working port(s) 122b defined by the distal tip portion 96 extends through a respective beveled face 214 formed at the distal tip portion 96 of the catheter 32. Alternatively, the distal tip portion 96 may be chamfered (not depicted) around a tangential perimeter 216 of the outer tangential surface 97 to define the beveled face(s) 214. In some embodiments, the proximal face 104 of the transparent cap 100 extends radially over the beveled face 214 to define an outlet 218 of the distal tip working port(s) 122b. Accordingly, for the distal head portion 34i as depicted, there is no cap irrigation port that passes through the transparent cap 100. Instead, irrigation ports 122b terminate the working channel 124 proximal to the transparent cap 100 and are configured to direct flow onto the proximal face 104 of the transparent cap 100.

In some embodiments, each of the illumination fiber optics 132 is disposed within a corresponding one of the distal tip working ports 122b, with the illumination fiber optic(s) extending into the transparent cap 100 of the distal head portion 34i. Each illumination fiber optic 132 may be configured to diffuse, refract, scatter, or otherwise redirect visible light 222 radially into the transparent cap 100. The transparent cap may also be configured to diffuse or scatter the visible light 222. The transparent cap 100 may contact a distal end portion 224 of the at least one illumination fiber optic 132, for example to effect the anchoring of the illumination fiber optic(s) 132 to the distal head portion 34. In some embodiments, an interface 226 between the distal end portion 224 of the illumination fiber optic 132 and the transparent cap 100 is configured to direct the visible light 222 radially away from the illumination fiber optic. For example, to augment redirecting the visible light 222, the distal end portion 224 of the illumination fiber optic(s) 132 may be uncladded. The redirection of the visible light 222 may occur along the entire length of the interface 226. In another example, the interface 226 includes a transparent or semi-transparent adhesive that scatters or refracts the visible light 222 away from the illumination fiber optic 132. In another example, the illumination fiber optic(s) 132 defines a relatively large numerical aperture (e.g., in a range of 0.35 to 0.65 inclusive). The example aspects above promote the redirection of the visible light 222 through the transparent cap 100.

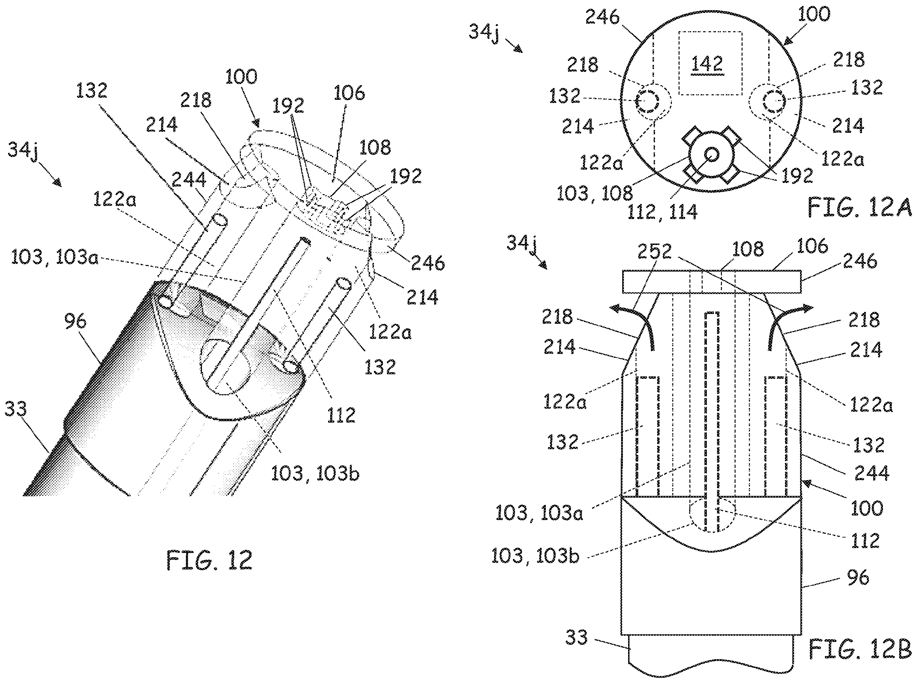

Referring to FIG. 12, a distal head portion 34j with recessed pressure reliefs 192 is depicted according to an embodiment of the disclosure. The distal head portion 34j includes many of the same components and attributes as the distal head portion 34i, which are identified with same-numbered reference characters. A distinction of the distal head portion 34j is that the pressure reliefs 192 extend proximally from the distal face 106 of the transparent cap 100. That is, the mouth 108 of the cap working port 103a is flush with the distal face 106 of the transparent cap 100. Another distinction of the distal head portion 34j is that the working ports 122 include the cap working ports 122a that extend into the transparent cap 100 but not through the distal face 106. Instead, the outlet 218 of the cap working port 122a extends through a radial face 244 of the transparent cap 100. In some embodiments, the beveled face 214 is formed in the radial face 244 of the transparent cap 100 to define the outlet 218. In some embodiments, each distal tip working port 122b is in fluid communication with a corresponding cap working port 122a. The transparent cap 100 may include a distal end portion 246 that extends radially over the cap working port 122a.

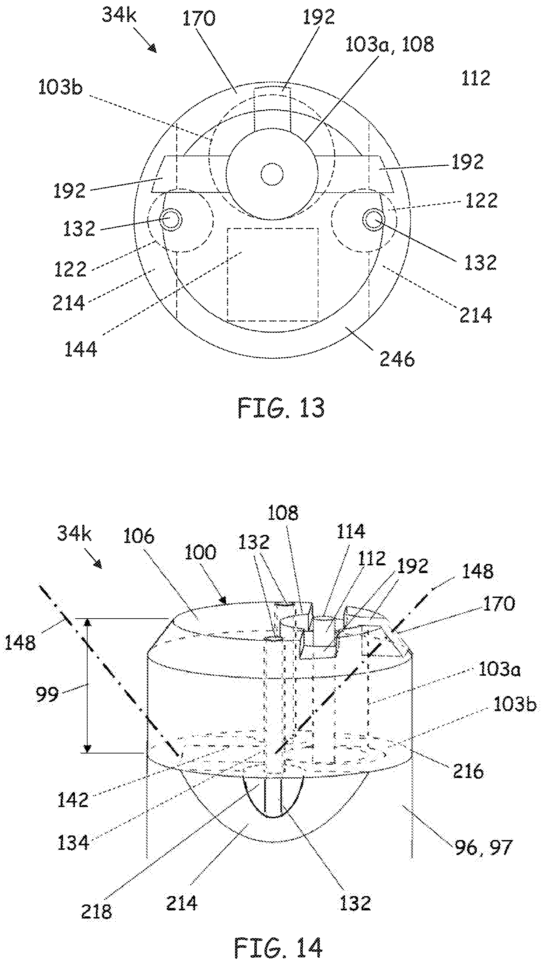

Referring to FIGS. 13 through 15, a distal head portion 34k with extended recessed pressure reliefs 192 is depicted according to an embodiment of the disclosure. The distal head portion 34k includes many of the same components and attributes as the distal head portion 34j, which are identified with same-numbered reference characters. A distinction of the distal head portion 34k is that pressure reliefs 192 extend radially to the outer tangential perimeter 170 of the distal face 106 of the transparent cap 100.

Functionally, the redirection of the visible light 222 away from the illumination fiber optic(s) 132 and into the transparent cap 100 can provide a more uniform irradiation of the target zone 56. The pressure relief(s) 192 of distal head portions 34i through 34k help stabilize the captured and targeted body stone 58 at the mouth 108 of the cap working port 103a in the aspiration mode. In the absence of the pressure relief(s) 192, the targeted body stone 58 can effectively plug the working port 103, creating a larger pressure differential across the body stone 58. The high pressure differential creates large forces that act on the targeted body stone 58. These large forces can cause, for example, the capture of the targeted body stone 58 to be unstable, such that the body stone 58 becomes dislodged from the working port 103. In another example, the large forces can cause the excessively large fragments of the targeted body stone 58 to become lodged in the working port 103 or to jam between the laser fiber optic 112 and the working port 103, thereby fouling the distal head portion 34 and damaging the laser fiber optic 112. The pressure relief(s) 192 enables aspiration flow around the captured body stone 58, thereby moderating the pressure differential across the body stone 58 and the attendant forces exerted on the body stone 58. The moderated pressures and forces mitigate capture instabilities and reduce the occurrence of excessively large fragments becoming lodged in the working port 103.

Arranging the transparent cap 100 to extend radially over the beveled portions 214 (FIGS. 10, 11, 14, 15, and 19) or, in the alternative, having the distal end portion 246 of the transparent cap 100 extend over the beveled portions of the distal end portion 246 (FIG. 12), deflects irrigation flow in a radial direction r to set up the flow field 256, as depicted at FIG. 15. The outlets 218 deliver irrigation flows 252 that vector radially outward, while an aspiration flow 254 draws flow into the mouth 108. In some embodiments, a peak outflow angle α of the irrigation flow 252 (i.e., the angle at which the maximum flux of irrigation flow occurs) is centered in the range of 10 to 90 degrees inclusive with respect to the central axis 110. In some embodiments, the peak outflow angle α is in a range of 10 to 60 degrees inclusive.

In operation, the radially outward facing outlets 218 create the flow field 256 that flows outward from the distal head portion 34k and folds inward toward the mouth 108. Flow for distal head portions 34i and 34j may behave in a similar manner. When the working channel 102 is used for aspiration, fragments of body stones 58 that are small enough (e.g., less than 0.5 millimeters) become entrained in the flow field 256 and evacuated through the mouth 108 and working channel 102. Other body stones 58 or fragments thereof that are too large to pass (e.g., 1 to 3 millimeters) are drawn into targeting proximity of the distal end 114 of the laser fiber optic 112 by the flow field 256. As these larger stones are brought into range of the laser fiber optic 112, the ablation laser system 46 may be energized to ablate the body stones 58. The ablation breaks the body stones 58 into smaller fragments that are then drawn into the working channel 102 through the mouth 108.

When a large body stone 58 enters or approaches the mouth 108 during aspiration, the working channel 102 may experience a drop in pressure as the stone obstructs the mouth 108. As such, in some embodiments, the ablation laser system 46 (FIG. 1) may be triggered by a pressure drop in the working channel 102 that is detected by the pressure sensor 48 of the aspiration system 44 to ablate the body stone 58 causing the blockage.

Functionally, establishing the flow field 256 to draw the body stone 58 toward the laser fiber optic 112 speeds up the process of laser lithotripsy. For example, when operating in the non-contact mode with peak outflow angles α that are within the 10 to 60 degree range, the irrigation flow 252 sweeps the small stones and stone fragments toward the mouth 108 of the aspiration channel 103 for more efficient operation. The irrigation flows 252 and the aspiration flow 254 may be continuous or pulsed, either individually or for both. In some embodiments, pulsed flows are synchronized with the laser pulses to enhance the ablation and removal of ablation particles. The need for hunting and chasing body stone 58 is reduced because the flow field draws the body stone 58 into an effective range (typically 0 to 3 millimeter) of the laser fiber optic 112. Also, having been drawn into the effective range of the fiber optic 112, the body stone 58 is more efficiently fragmented by the ablation process. Navigation within the target zone 56 is improved because the redirection of some of the visible light 222 provides a more uniform lighting of the target zone 56. The amount of attenuation by the smaller fragments and particles from the body stones 58 in the field of view 148 is reduced by the aspiration and by the presence of the transparent cap 100 in the near field of view 148.

Referring to FIGS. 16 through 19A, distal head portions 34l and 34m are depicted according to embodiments of the disclosure. The distal head portions 34l and 34m may include many of the same components and attributes of the other distal head portions 34 described above, some of which are indicated with same-numbered reference characters. Distinctions of the distal head portion 34l include a single illumination fiber optic 132, the transparent cap 100 having a convex or domed profile 262, distal tip working ports 122b defining an asymmetric flow cross-section 264, and the laser fiber optic 112 being supported by a laser fiber optic port 266 that is offset from the cap working port axis 111.

The single illumination fiber optic 132 may be configured to exert both a pulling force and a pushing force on the distal head portion 34l. In some embodiments, the cross-section of the single illumination fiber optic 132 measures 0.2 millimeter x 0.5 millimeter.

Functionally, the single illumination fiber optic 132 may occupy less cross-section of the distal head portion 34l than do a pair of illumination fiber optics 112 of, for example, the distal head portion 34d of FIG. 5. In addition to having less fiber optic cross-section, the associated structural cross section required for anchoring the fiber optics (i.e., structure to which the fiber optic(s) is bonded) is also reduced. The reduction in cross-section provides more area for other components of the distal head portion 34l (e.g., for working ports 103, 122b), or a reduction in the overall cross-section of the distal head portion 34l, or a combination of both. For example, in one embodiment, the maximum outer dimension OD1 is in the range of 2 to 2.5 millimeters inclusive and the minimum outer dimension OD2 is in the range of 1.7 to 2 millimeters inclusive, while still providing an increased cross-sectional flow area relative to other embodiments.

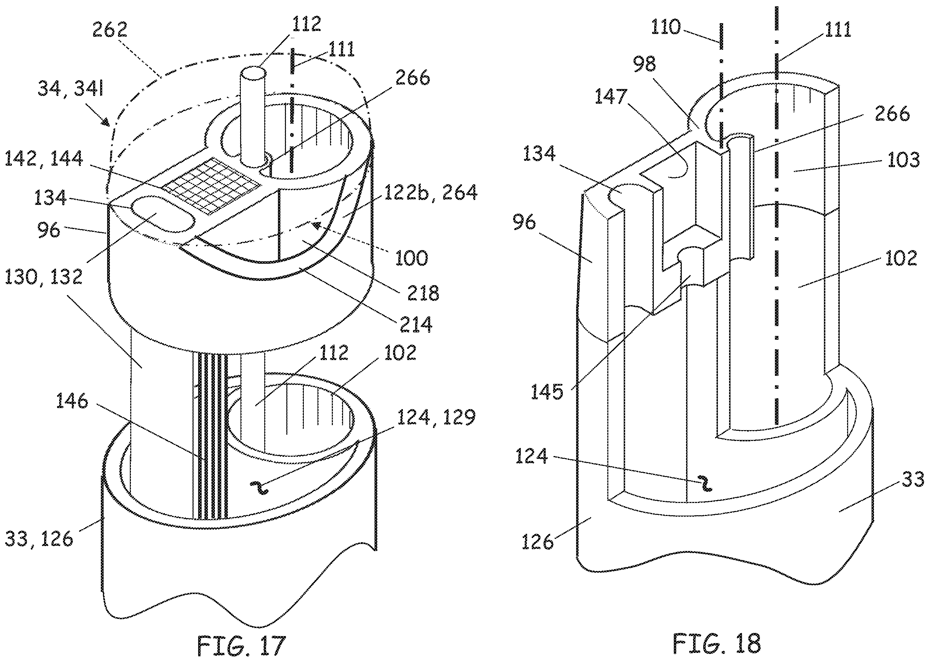

The domed profile 262 of the transparent cap 100 may be generally hemispherical and define the cap working port 103a therethrough. In some embodiments, the distal head portion 34l is oblong, defining the major and minor axes 171 and 169 and attendant outer dimensions OD1 and OD2, akin to distal head portion 34h (FIG. 9). In some embodiments, the domed profile 262 is asymmetrical. For the depicted distal head portion 34l, the domed profile 262 is asymmetrical along the major axis 171 (FIG. 16A) while being symmetrical along the minor axis 169 (FIG. 18). The domed profile 262, as depicted, defines a maximum axial dimension Z that is parallel to the central axis 110 of the distal head portion 34l. In some embodiments, the maximum axial dimension Z of the domed profile 262 is located over the imaging receiver 142. The distal head portion 34l may also include the pressure reliefs 192 recessed into the domed profile 262.

Functionally, the domed profile 262 of the transparent cap can provide smooth and easy passage of the distal head portion 34l through body vessels such as the ureter and calyces, particularly when steering the distal head portion 34l through a turn. Arranging the maximum axial dimension Z of the transparent cap 100 to be in line with the imaging receiver 142 increases the length (and therefore the clarity) of the path normal to the imaging receiver relative to the flat distal face 106 of other transparent caps 100 (e.g., FIGS. 2A, 3A, and 3B). The convexity of the domed profile 262 may also be configured to act as a lens to magnify the image as viewed by the imaging receiver 142. The pressure reliefs 192 function as described attendant to FIGS. 13 and 14.

The asymmetric flow cross-section 264 of the distal tip working ports 122b may be configured to occupy a greater fraction of the cross-sectional area of the distal head portion 34l than for axisymmetric working ports such as the circular working ports 122 of the distal head portion 34b or the oblong working ports 122 of distal head portions 34c, 34g, 34h. Effectively, structure is provided in the distal tip portion 96 for bounding the working port 103 and for mounting the laser fiber optic 112, the illumination fiber optic 132, and the imaging receiver 142. The balance of the oblong cross-section 167b of the distal head portion 34l is structured to provide the asymmetric flow cross-sections 264.

The laser fiber optic port 266 protrudes radially into the working port 103 and may be dimensioned to provide a close sliding fit with the laser fiber optic 112. The working port 103 defines a maximum inner radius R. The protrusion of the fiber optic port 266 encroaches on the maximum inner radius R to define a minimum inner dimension 268 of the working port 103. The laser fiber 112 may be mounted within the port 266 during manufacturing and sterilized together with the catheter 32. Various methods of mounting the laser fiber can be used, including (but not limited to) friction-controlled mechanical attachment, over-molding, adhesive bonding, or other suitable techniques. Such pre-integration of the laser fiber into the scope reduces the preparation time for surgery, as the surgeon does not need to insert the fiber into a scope.

The distal end 114 of the fiber 112 may be recessed within the working port 103 proximal to the surface distal 106 to mitigate fiber burn back effects.

Functionally, the asymmetric flow cross-sections 264 act to increase the flow cross-sections of the distal tip working ports 122b relative to a circular, oblong, or other axisymmetric cross-section, providing, for example, greater cross-section for irrigation flow or passage of catheter tools. Likewise, the offset of the laser fiber optic port 266 and laser fiber optic 112 provides a greater unimpeded flow cross-section for the working port 103. That is, for a working port 103 having a given cross-sectional flow area, a minimum inner dimension 265 (FIG. 7) for arrangements having the laser fiber optic 112 substantially centered within the working port 103 (e.g., FIGS. 2 through 9 as depicted) is somewhat less than an inner radius of the working port 103, whereas the minimum inner dimension 268 of the working port 103 of the distal head portion 34l may be substantially greater than the maximum inner radius R of the working port 103 (FIG. 16). For embodiments where the working port 103 and mouth 108 serves as an aspiration inlet, the larger minimum inner dimension enables the aspiration of larger stone fragments from the target zone 56 than for concentrically positioned laser fiber optics 112. Furthermore, the laser fiber optic port 266 can provide additional protection of the laser fiber optic 112 from damage due to passage of stone fragments at the constriction of the working port 103 where the minimum inner dimension 268 is defined.

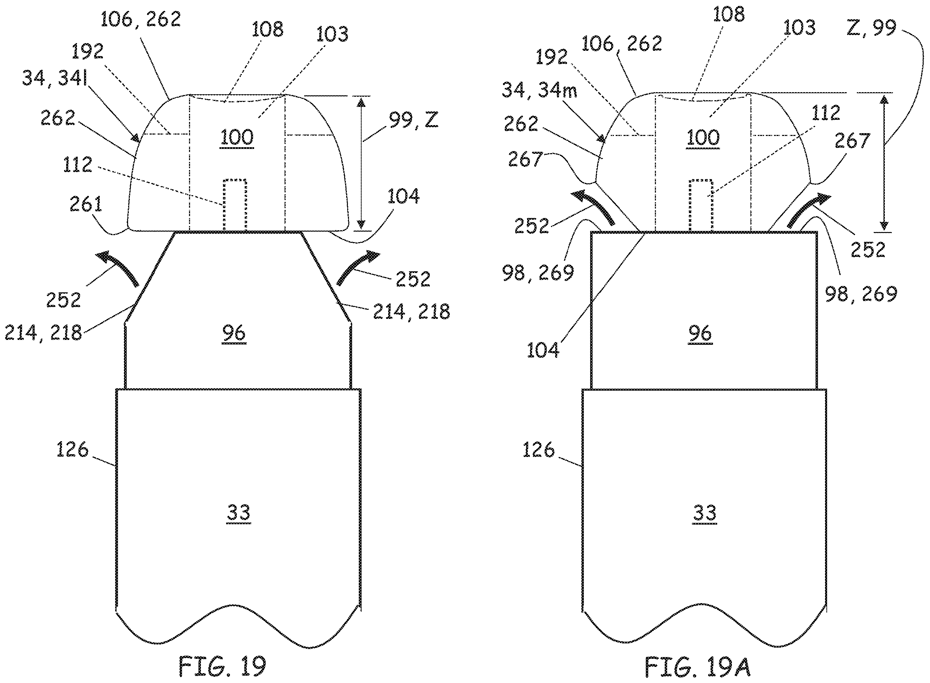

The distal head portion 34l depicts the transparent cap 100 as extending radially over the beveled portions 214 of the distal tip portion 96, akin to FIGS. 10, 11, 14, and 15 discussed above. The transparent cap 100 may include a transition 261 between the proximal face 104 and the domed profile 262. The transition 261 may be, for example, arcuate (depicted) or chamfered. The transition 261 can enable smooth movement of the catheter 32 in the proximal direction (e.g., during removal through body vessels). Alternatively or in addition, a bevel or bevels 267 may be defined on the transparent cap 100, as depicted for distal head portion 34m at FIG. 19A. The bevels 267 (or, alternatively, a chamfer) on the transparent cap 100 has the effect of vectoring the irrigation flows 252 radially outward. In some embodiments, the distal tip portion 96 defines outlets 269 that are co-planar with the distal face 98 of the distal tip portion 96 (depicted). Embodiments where radially outward facing outlets 218 are combined with bevels 267 are also contemplated.

Referring to FIGS. 20 and 20A, a distal head portion 34n is depicted according to an embodiment of the disclosure. The distal head portion 34n may include many of the same components and attributes as other distal head portions 34 described herein, some of which are indicated with same-numbered reference characters. A characteristic of the distal head portion 34n is that the distal tip portion 96 includes an extension portion 286 that extends from a base platform 288 to the distal face 98. The working port 103 extends through the extension portion 286 and distal face to define the mouth 108 at the distal face 98. In some embodiments, the extension portion 286 includes a reducing flange 290 protrudes radially inward to define the mouth 108. The reducing flange 290 defines a diameter of the mouth 108 that is less than an inner diameter of the working port 103 proximal to the reducing flange 290. In some embodiments, the reducing flange 290 decreases the area of the mouth 108 by 5% to 50% relative to the area of the working channel 102 proximal to the reducing flange 290.

The reducing flange 290 may also be implemented with distal head portions 34 where the mouth 108 is defined by the transparent cap 100. A transparent cap 100 with the reducing flange 290 is depicted at FIG. 2A, and may be implemented *mutatis mutandis* to any of the transparent caps 100 disclosed herein.

A maximum axial offset Δ of the imaging receiver is defined as a distance from a distal extremity 291 of the extension portion 286 to the imaging receiver 142, the distance being parallel to the working port axis 111. For embodiments where the distal face 98 defines a plane 292 that is normal to the working port axis 111 (depicted in FIGS. 20A and 21A), the distal extremity 291 of the extension portion 286 is any point on the plane 292 and the maximum axial length Δ is a distance from the plane 292 to the imaging receiver 142 that is parallel to the working port axis 111. For embodiments where the distal face 98 is a contoured surface (e.g., akin to the domed profile 262 of the transparent cap 100 of distal head portions 34l and 34m in FIGS. 16A, 17, 19 and 19A), the distal extremity 291 of the mouth 108 may be singular. An example of a singular distal extremity on the transparent cap 100 of distal head portion 34l is identified with reference character 291' in FIG. 16A_{.} In some embodiments, the maximum axial length Δ is within a range of 1 millimeter to 10 millimeters inclusive. In some embodiments, the maximum axial length Δ is within a range of 1 millimeter to 5 millimeters inclusive.

The distal end 114 of the laser fiber optic 112 is positioned proximate the mouth 108. An axial location δ of the distal end 114 of the laser fiber 112 is defined relative to a distal-most location 292 of the mouth 108. For embodiments where the mouth 108 defines the plane 292 normal to the working port axis 111 (depicted in FIGS. 20A and 21A), the distal-most location 292 is any point on the plane 292 and the axial location δ is the distance along the working port axis 111 from the plane 292. For embodiments where the mouth 108 is defined on a contoured surface (e.g., such as with the domed profile 262 of the transparent cap 100 of distal head portions 34l and 34m in FIGS. 16A, 17, 19 and 19A), the distal-most location 292 of the mouth 108 may be singular, such as identified in FIGS. 16A. Where the distal-most location 292 is singular, the axial location δ is defined as distance between the distal end 114 of the laser fiber and the distal-most location 292 that is parallel to the working port axis 111.

In some embodiments, the positioning of the distal end 114 of the laser fiber optic 112 is selective over a range of axial locations δ. In some embodiments, the distal end 114 of the laser fiber 112 can be selectively positioned (i.e., is "selectively positionable") at axial distances ranging from 1 millimeter distal to the distal-most location 292 to 3 millimeters proximal to the distal-most location 292 (inclusive). In some embodiments, the axial locations δ range from flush with the distal-most location 292 to 1 millimeter proximal to the distal-most location 292 (inclusive). In some embodiments, the axial locations δ range from 0.05 millimeter to 0.6 millimeter inclusive proximal to the distal-most location 292.

The recess 147 for holding the imaging receiver 142 is formed on the base platform 288 and is arranged to face in distally. In some embodiments, the distal face 98 and the base platform 288 define substantially parallel planes (depicted). In some embodiments, a shoulder 294 transitions between the outer tangential surface 97 of the distal tip portion 96 and the base platform 288 at the tangential perimeter 216. Likewise, a shoulder 296 transitions between a tangential surface 298 of the extension portion 286 and the distal face 98. The shoulders 294, 296 may be, for example, arcuate (depicted), radiused, or beveled.

The pressure relief(s) 192 extend axially from the distal face 98 and radially through the extension portion 286 and outer tangential surface 97. The pressure relief(s) 192 may be a notch or notches. The cross-sectional size of the notches can be from 0.1 to 1 millimeters inclusive in axial depth and 0.2 to 0.5 millimeters inclusive in tangential width. The function of the pressure relief(s) 192 is described above attendant to FIGS. 10 through 15.

Referring to FIGS. 21 through 21C, a distal head portion 34o is depicted according to an embodiment of the disclosure. The distal head portion 34o includes various components and attributes as the distal head portion 34n, some of which are indicated by same-numbered reference characters. In addition, the distal head portion 34o includes distal tip working ports 122b that extend through the distal tip portion 96 and are in fluid communication with the working channel 124, which is used for irrigation. The distal tip working ports 122b may be configured to direct the irrigation flow 252 through the base platform 288 or the tangential surface 97 of the distal tip 96. The outlets of the distal tip working ports 122 may define an outlet angle ϕ relative to the working port axis 111 in the distal direction 50 for directing the irrigation flow 252. In some embodiments, the outlet angle ϕ is within a range of 0 degrees to 170 degrees inclusive relative to a distal direction along the central axis 110. In some embodiments, the outlet angle ϕ is within a range of 10 degrees to 70 degrees inclusive. In some embodiments, the outlet angle ϕ is within a range of 20 degrees to 45 degrees inclusive.

In some embodiments, the laser parameters for treatment with the various disclosed embodiments herein are be selected in accordance with the following guidelines:
(1) A wavelength in the range of 1.9 -2.1 micrometers to match peak of water absorption which is a major initial chromophore for body stone ablation.
(2) Limiting pulse energy to prevent stone retropusion effects so as not to overcome the effects of aspiration and propel the treatment stone away from the opening of the aspiration working port 103. The laser pulse energy for stone dusting may be minimal as low as 0.001 Joules to 0.2 Joules for this purpose. For stone fragmentation, the laser pulse energy may be in the range 0. 2 Joules to 2 Joules inclusive.
(3) For simultaneous aspiration and irrigation applications, the heat energy absorbed by the liquid medium within a body organ may be partially or completely evacuated due to the aspiration. For aspiration flows 254 in a range of 50 to 100 milliliters per minute inclusive and irrigation flows 252 in a range of 10 to 150 milliliters per minute inclusive, the average laser power delivered to the target zone 56 by the ablation laser system 46 may be increased over conventional laser lithotripsy techniques without adverse effects. The maximum average power for ureteral applications can be as high as 30 to 50 Watts inclusive; for kidney applications, 60 to 120 Watts inclusive; for bladder applications, up to 200 Watts inclusive. These average powers represent an increase that is several times greater than with the conventional laser lithotripsy techniques, and without increasing temperature of the liquid medium beyond critical levels for the ureter, kidney or bladder. For example, conventional laser lithotripsy is typically limed to 10 to 30 Watts in for ureteral applications and 30 to 50 Watts in kidney application. The proposed average laser power increases thus represent an increase that is 1.5 to 2.5 times greater than conventional systems. The increase in the average laser power (or in the pulse repetition rate for fixed laser pulse energy systems) increases the speed of ablation proportionally.

Functionally, endoscopic systems 30 implementing the distal head portion 34n operate in similar manner to endoscopic systems 30 utilizing the distal head portion 34a (i.e., where the aspiration and irrigation occur sequentially using the working channel 102 as a common working channel 109). Endoscopic systems 30 implementing the distal head portion 34o operate in similar manner to endoscopic systems 30 that implement simultaneous aspiration and irrigation (e.g., with distal head 34b). For both the distal heads 34n and 34o, the maximum axial offset Δ between the imaging receiver 142 and the distal extremity 291 of the extension portion 286 enables the mouth 108 to be disposed within the viewing angle β of the imaging receiver 142. Being within the viewing angle β does not necessarily mean that the mouth can be visualized by the visualization system 54, but only that at least a portion of the mouth 108 falls within the viewing angle β of the imaging receiver 142. For embodiments where the mouth 108 is supported by an opaque structure (e.g., the extension portion 286 is made of an opaque polymer or rubber), the mouth 108 may not be visible. Where the mouth 108 is obscured by an opaque structure, the target zone 56 is still mostly visible, and the reaction of the body stones 58 or fragments thereof to the ablation process and the flow field 256 can be monitored. For embodiments where the mouth 108 is supported by a transparent or semi-transparent medium (e.g., the transparent cap 100 of distal head portions 34a through 34m), the mouth will be visible through the medium, which enables complete visualization of the ablation process.

In contrast with conventional ureteroscopes, the distal face 98 of the disclosed distal head portions 34 is designed to be in contact or quasi contact with the targeted stone 58 or fragment. For axial locations δ greater than about 0.2 millimeters proximal to the mouth 108, the distal end 114 of the laser fiber optic 112 is not always in direct contact with the body stone 58 or stone fragment, even during active aspiration. Despite instances of a lack of direct contact, laser energy can be effectively delivered to the stone 58 in the liquid medium environment through a distance of up to about 3 millimeters. By operating the laser at wavelengths that are at or near peak absorption for water, the water initially absorbs the laser energy to quickly form a vapor channel between the distal end 114 of the laser fiber 112 and the stone material, greatly reducing the attenuation of the laser energy. Also, the stone 58 or fragment may oscillate or rotate at the mouth 108 so that the surface of the stone 58 or fragment moves perpendicular to the axis of the laser fiber 112. Such oscillation and rotation increases the speed of ablation. The phenomena and effects of vapor channeling and laser fiber oscillation are described in further detail at International Patent Application No. PCT/US19/42491 to Altshuler, et al., incorporated by reference above.

The reducing flange 290 acts to prevent blockage of the working channel 102 and working port 103. During aspiration, some fragments generated during ablation will have a dimension that is equal to or larger than the inner diameter of the working channel 102. The presence of the laser fiber 112 reduces the flow cross-section of the working channel 102, such that the fragment becomes lodged between the laser fiber 112 and the working channel 102. The reduced area of the mouth 108 when defined by the reducing flange 290 acts to reduce the size of the fragments that can pass into the working channel 102, thereby reducing the incidence of blockage.

The different outlet angles ϕ of distal head portion 34o are suitable for different operating modes. In contact mode operation, used to ablate large stones or stone fragments, irrigation flows 252 should be directed so as not to impinge on the larger stones or fragments. Accordingly, distal tips 96 defining outlet angles ϕ in a range of 20 degrees to 170 degrees inclusive may be utilized. In non-contact mode, the irrigation flows 252 maintain churning of small fragments within the target zone 56. Accordingly, distal tips 96 defining outlet angles ϕ in a range 20 degrees to 45 degrees inclusive may be utilized.

When operating the working channel 102 in aspiration, the suctioning of the fragments towards the working channel may partially or completely overcome the retropulsion effect in contact mode and accelerate treatment of small fragments in non-contact mode. The disclosed endoscopic systems 30 operate efficiently when laser operates in dusting mode, where the ablated particles that are smaller than the inner dimension of the working channel 102 can be evacuated from human body by aspiration to provide a stone-free treatment result. For example, a SUPERPULSE Thulium fiber laser with pulse energy from 0.02 to 1 J can provide fragmentation and dusting ablation for particle sizes below 0.5 millimeters. If the laser fiber 112 has core diameter in a range of 0.05 to 0.2 millimeters and an outer diameter below 0.4 millimeters, and the inner diameter of the working channel 102 is greater than 1 millimeter, the particles having dimensions less than 0.5 millimeters can be evacuated through the working channel 102.

When performing a laser lithotripsy procedure, aspiration flows 254 of approximately 200 milliliters per minute may be utilized. The aspiration generally produces a negative pressure within a kidney. Such negative pressure should not deviate from the surrounding environmental pressure by more than 20%.

Operationally, the aspiration flow 254 and irrigation flows 252 may be balanced to maintain a net positive irrigation flow. In some embodiments, the irrigation flow 252 exceeds the aspiration flow 254 by up to 50 milliliters per minute. In some embodiments, the net positive irrigation flow is in a range of 10 to 30 milliliters per minute inclusive.

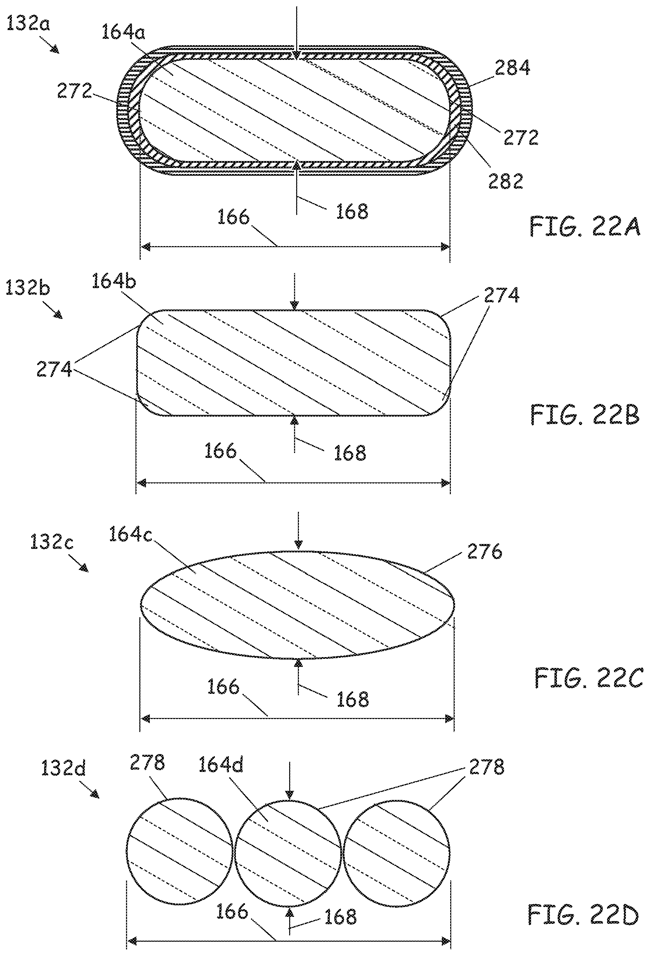

Referring to FIGS. 22A through 22D, proposed oblong cross-sections 164a through 164d for illumination fiber optics 132a through 132d are depicted according to embodiments of the disclosure. Herein, illumination fiber optics 132 and their respective oblong cross sections 164 are referred to collectively and generically by reference characters 132 and 164, respectively, and specifically by reference characters 132 and 164 followed by a letter suffix (e.g., illumination fiber optic 132a with oblong cross section 164a). Example and non-limiting cross-sections 164 include: a generally rectangular shape with semicircular ends 272 ("obround" cross-section 164a of illumination fiber optic 132a of FIG. 22A); a generally rectangular shape with radiused corners 274 ("rounded rectangular" cross-section 164b of illumination fiber optic 132b of FIG. 22B); a generally elliptical shape 276 (cross-section 164c of illumination fiber optic 132c of FIG. 22C); and a plurality or bundle of illumination fibers 132d having circular shapes 278 that combine to define a ribbon (combined cross-section 164d of illumination fiber optics 132d). For the cross-section 164d, the bundle of illumination fibers 132d may be arranged so the circular shapes 278 are sequential in the tangential direction θ about the central axis of the catheter 32 at the distal head portion 34. In some embodiments, the bundle of illumination fiber optics 132d may be centered about a plane (depicted).

The illumination fiber optics 132 may also include a buffer layer 282 and an overcoat layer 284 (FIG. 22A). In some embodiments, the buffer layer 282 is, for example, an FPL-9 layer having a thickness that is within a range of 10 - 20 micrometers inclusive. In some embodiments, the overcoat layer 284 is, for example, a fluoropolymer such as blue TEFZEL^{®} having a thickness that is within a range of 20 - 50 micrometers inclusive. While the coating layers 282 and 284 are depicted for illumination fiber optic 132a of FIG. 22A, it is understood that the coating layers 282 and 284 may be incorporated with any illumination fiber optic 132, including illumination fiber optics 132b, 132c, and 132d of FIGS. 22B through 22D. In some embodiments, the major dimension 166 of the laser fiber optic 132 is in a range of 0.2 to 2.0 millimeters inclusive. In some embodiments, the minor dimension 168 is in a range of 0.1 to 1.0 millimeters inclusive. In one embodiment, the major dimension 166 of the laser fiber optic 132 is 0.6 millimeters and the minor dimension is 0.2 millimeters. In some embodiments, a ratio of the major dimension to minor dimension is in a range of 2:1 and 5:1 inclusive.

Functionally, the oblong cross sections 164 of the illumination fiber optics 132 enable the sectional dimensions of the catheter 32 and distal head portion 34d to be reduced relative to the distal head portion 34a. The oblong cross sections 164 can be arranged to provide a lower profile in the radial direction while increasing the dimension (and stiffness) in the tangential direction. The overcoat layer 284 provides protection for the cladding layer 282 as well as lubricity for ease of sliding the illumination fiber optic 132 within the lumen 107 during steering operations. In some embodiments, the overcoat layer extends proximate to but not through the distal head portion 34. For the illumination fiber optic 132d, the overcoat layer 284 may also hold the individual circular fiber optics together to bind together and stabilize the oblong cross-section 164d of the ribbon.

In addition to acting as an optical waveguide that transmits visible light, each oblong cross-section 164 provides enhanced rigidity along the major dimension 166 of the illumination fiber optic 132 (i.e., along the tangential direction θ), while enabling and facilitating flexing of the oblong cross-section 164 along the minor dimension 168 (i.e., along the radial coordinate r perpendicular to the major dimension 166). Accordingly, the oblong cross-sections 164 of the illumination fiber optics 132 provide torsional rigidity for catheter 32 having a flexible shaft, partially or totally negating the need for a separate torsion sleeve that is customary in conventional flexible catheters.

Accordingly, utilizing illumination fiber optics 132 that define oblong cross sections 164 enables the elimination of a torsion sleeve and pull wires and associated connectors. As a result, the radial profile of the distal head portion 34d can be diminished for reducing the invasiveness and enhancing the safety of the laser lithotripsy procedure.

Referring to FIG. 23, a steering handle 300 for use as the handle 38 is depicted according to an embodiment of the disclosure. The steering handle 300 may be implemented, for example, for catheter shafts 33 that are flexible. The steering handle 300 is coupled to the catheter 32 and to a pair of illumination fiber optics 132, and may be configured to exert forces on the illumination fiber optics 132 for articulation of the distal head portion 34. In some embodiments, the steering mechanism 39 of the steering handle 300 includes a rotating cam 310 directly coupled to the illumination fiber optics 132. Example embodiments of suitable steering handles are further described at U.S. Provisional Patent Application No. 62/868,271, filed June 28, 2019, and at U.S. Provisional Patent Application No. 62/868,105, filed June 28, 2019, both owned by the assignee of the present application and the contents of which are hereby incorporated by reference herein in their entirety except for express definitions and patent claims contained therein.

The illumination fiber optics 132 may be affixed to the rotating cam 310, for example, with a bonding adhesive 312 (depicted). The steering mechanism 39 may also include a shaft 316 about which the rotating cam 310 rotates. In some embodiments, the steering mechanism 39 includes a thumb lever 318 coupled to the rotating cam 310. In some embodiments, the illumination fiber optics 132 are routed from the illumination system 52 to the rotating cam 310, from the rotating cam 310 to routing sheaths 320, and from the routing sheaths 320 to the distal head portion 34 via the catheter shaft 33. In some embodiments, the illumination system 52 includes a light emitting diode 322 as the visible light source. In some embodiments, the illumination system 52 is housed within the steering handle 38, being powered by one or more batteries 324 (depicted).

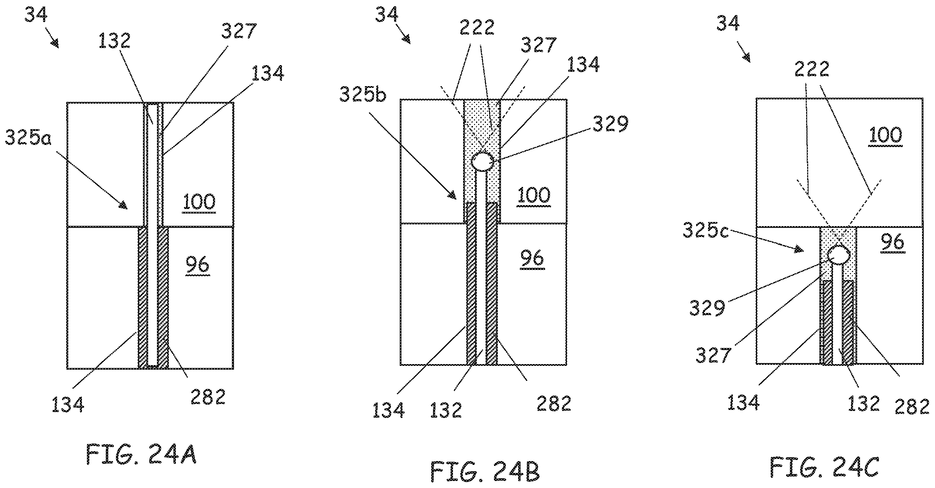

Referring to FIGS. 24A through 24C, terminations 325 for anchoring the illumination fiber optics 132 to the distal head portion 34 is depicted according to embodiments of the disclosure. The terminations are referred to collectively and generically with reference character 325 and individually and specifically by reference character 325 followed by a letter suffix (e.g., "termination 325a"). For the termination 325a (FIG. 24A), a straight illumination fiber optic 132 is routed into the fiber optic port 134 and bonded to the transparent cap 100 with a transparent or semi-transparent bonding adhesive 327. In some embodiments, the buffer layer 282 is stripped from the portion of the fiber optic that is inserted into the transparent cap 100.

For termination 325b (FIG. 24B), a termination head 329 is formed at the distal end of the illumination fiber 132. The termination head 329 is depicted as a sphere in FIG. 24B, but is more generally characterized as having a radial dimension that is greater than the radial dimension of the shaft of the illumination fiber optic 132, and having rounded surfaces. The termination head 329 is potted within the fiber optic port 134 defined by the transparent cap 100, using the transparent or semi-transparent bonding adhesive 327.

For termination 325c (FIG. 24C), the termination head 329 is potted within the fiber optic port formed only in the distal tip portion 96 of distal head portion 34, again using the transparent or semi-transparent bonding adhesive 327. The transparent cap 100 extends over the distal end of the fiber optic port 134

Functionally, the effect of stripping the buffer 282 is to enhance redirection of the visible light 222, as discussed above. The refraction of the visible light 222 through the rounded surfaces of the termination head 329 provides greater divergence of the beam where mismatch of the refractive indices between the illumination fiber optic 132 and the bonding adhesive 327 may be present. The larger dimension of the termination head 329 relative to the dimension of the shaft of the illumination fiber optic 132 also provides structural integrity to the anchoring at the terminations 325b and 325c.

In operation, a first of the illumination fiber optics 132 is pulled in tension when the rotating cam 310 is actuated in a first rotational direction 326 to articulate the distal head portion 34 in a first lateral direction. A second of the illumination fiber optics 132 is pulled in tension when the rotating cam 310 is actuated in a second rotational direction 328 to articulate the distal head portion 34 in a second lateral direction.

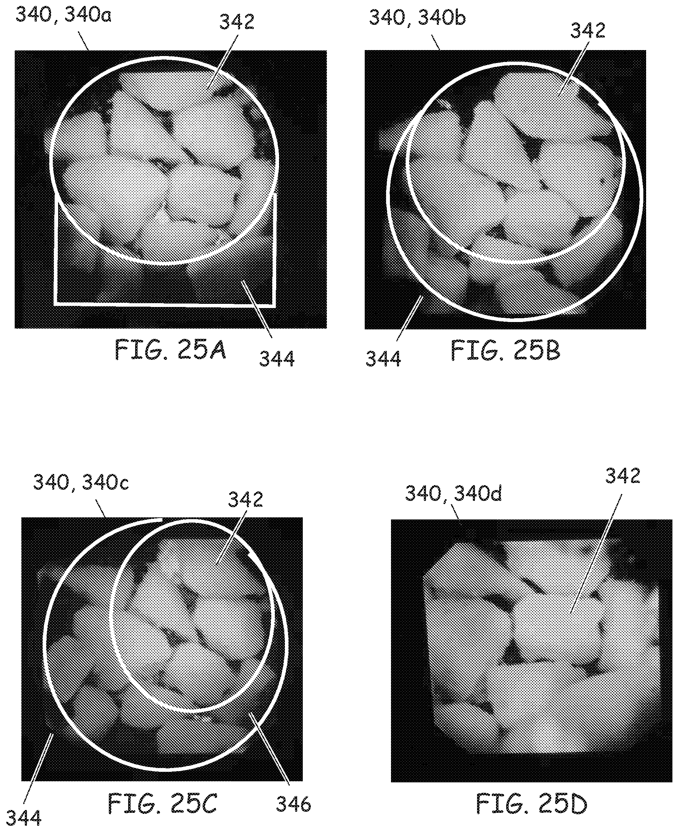

Referring to FIGS. 25A through 25D, images 340 of the target zone 56 as produced by the visualization system 54 for various configurations of the distal head portion 34j are presented. Herein, the images 340 are referred to collectively and generically by reference character 340 and individually or specifically by reference character 340 followed by a letter suffix (e.g., image 430a). Image 340a of the target zone 56 for the distal head portion 34j without the transparent cap 100 (i.e., an axial cap thickness 99 of zero) is presented at FIG. 25A. The image 340a exhibits a dark shadow fringe 344 along the lower edge.

Image 340b (FIG. 25B), viewed through an axial cap thickness 99 of 1 millimeter, reduces the dark shadow fringe 344 relative to the image 340a, and exhibits a focused and illuminated zone 346 that transitions between the focused and well illuminated zone 342 and the dark shadow fringe 344, providing a more uniform illumination relative to image 340a. Image 340c (FIG. 25C), viewed through an axial cap thickness 99 of 1.25 millimeter, further reduces the dark shadow zone 344. Image 340d (FIG. 25D), viewed through an axial cap thickness 99 of 1.5 millimeter, provides an image that is substantially uniformly lit.

Images 340 demonstrate that as the axial cap thickness 99 increases, the illumination light is spread out to more uniformly irradiate the targeted zone 56 as viewed by the visualization system 54. At some point, for still greater axial cap thicknesses 99, as well as for greater maximum axial offsets Δ of distal head portions 34o and 34p (FIGS. 16A and 17A), the separation between the imaging receiver 142 and the distal face 106 may cause an unacceptable dimming of the image. Accordingly, in some embodiments, the range of the axial cap thickness 99 is between 1 and 10 millimeters inclusive; in some embodiments, the range of the axial cap thickness 99 is between 1.2 and 5 millimeters inclusive.

For the images 340b, 340c, and 340d, the mouth 108 of the distal head portion 34j is in the field of view 148. Surprisingly, the presence of the mouth 108 and the working port 103 leading to the mouth 108 introduce little or no distortion to the images 340b, 340c, and 340d, despite the presence of the extensive structure of the extension 182 and the pressure reliefs 192 (FIGS. 10 and 11). The other disclosed configurations for the transparent cap 100, having less structure than with distal head portion 34j, may also introduce little or no distortion to the image.

In some embodiments, the foregoing methods of operation are provided as instructions on a tangible, non-transitory medium that are supplied with the catheter 32. Non-limiting examples of a tangible, non-transitory medium include a paper document and computer-readable media including compact disc and magnetic storage devices (e.g., hard disk, flash drive, cartridge, floppy drive). The computer-readable media may be local or accessible over the internet. The instructions may be complete on a single medium, or divided among two or more media. For example, some instructions may be written on a paper document that instruct the user to access one or more of the steps of the method over the internet, the internet-accessible steps being stored on a computer-readable medium or media. The instructions may be in the form of written words, figures, and/or video presentations.

### EXAMPLE 1

The distal portion 35 of a prototype for the catheter 32 was constructed using a transparent cap 100 fabricated from quartz according to the embodiment depicted at FIGS. 13 through 15. The transparent cap 100 of this embodiment was attached to a distal tip of a conventional ureteroscope having an outer diameter of 3 millimeters at the distal tip, an imaging receiver 142 having dimensions of 1 x 1 millimeter, a working channel 102 with inner diameter 1.2 millimeter and terminated in same plane as input of imaging receiver 142. The outer diameter OD of the transparent cap 100 was 3 millimeters, with an axial cap thickness 99 of 2 millimeters. The inner diameter of the cap working port 103a was 0.8 millimeter and two notches 194, each 0.3 millimeter wide, extended to the outer tangential perimeter 170 of the distal face 106 to serve as the pressure reliefs 192. Illumination light was delivered through two illumination fiber optics 132 having a core diameter of 0.12 millimeter and a numerical aperture 0.6 which delivered visible light from an LED at powers not exceeding 0.1 Watt. The laser fiber 112 used for stone ablation had a core diameter 0.2 millimeter, an outer diameter of 0.38 millimeter, and a numerical aperture of 0.22. The distal end 114 of the laser fiber 112 was positioned fully inside the working port 103a at a distance of 0.2 millimeter proximal to the mouth 108. In the Example 1 configuration, the working channel 102 was used for aspiration and irrigation was delivered through the hollow 129 of the shaft 33 of the conventional ureteroscope, as described attendant to FIG. 3B.

A SUPERPULSE Thulium fiber laser (FiberLase U2, with wavelength 1940 nm and peak power 500 Watts, manufactured by IPG Photonics of Oxford, Massachusetts, U.S.A.) operating at a pulse energy of 0.1 Joules, a pulse repetition rate 300 Hz, and an average power of 30 Watts was used for ablation of stones in all experiments. As a model of body stones, phantoms made out of BEGOSTONE material (universally accepted model of body stones) were utilized. Treatment simulation was conducted in a cuvette filled with water. Five phantom stones of about 1.5 millimeter diameter each were used for the simulation; weights and times were precisely measured, but dimensions of the phantom stones were approximate.

Comparison was made between the Example 1 configuration and a conventional configuration operating with the working channel 102 delivering irrigation fluid. For the conventional configuration, the cap was removed so that the end of the catheter shaft was exposed. The laser fiber was positioned so that the distal tip extended 3.5 millimeters beyond the end of the shaft. For the Example 1 configuration, completion of treatment was defined as ablation of the stone samples to particles that completely evacuated through the aspiration channel. For the conventional configuration, the treatment completion was defined as breakage of stone samples to particles smaller than 0.5 millimeter (which were removed with an aspiration flow of 10 milliliters/minute at a distance of about 40 centimeters). The results are summarized in Table 1.

**Table 1. Efficiency of stone breaking for conventional vs. disclosed configuration**

| *Mode of treament* | *Configuration* | *Irrigation flow, ml*/*min* | *Aspiration flow, ml*/*min* | *Initial stone weight, mg* | *Total laser time ON, s* | *Stone breakage rate, mg*/*s* |
|---|---|---|---|---|---|---|
| Contact | Conventional | 10 | 0 | 223 | 960 | 0.23 |
| | Example 1 | 100 | 100 | 260 | 250 | 1.04 |
| Non-contact | Conventional | 10 | 0 | 28 | 47 | 0.6 |
| | Example 1 | 100 | 100 | 27 | 12 | 2.3 |

As can be seen from Table 1, the Example 1 configuration provides more than a fourfold increase in the efficiency of stone breaking in the contact mode and more than a 3.5-fold increase in non-contact mode compared to the conventional configuration without increase in laser power required.

Each of the additional figures and methods disclosed herein can be used separately, or in conjunction with other features and methods, to provide improved devices and methods for making and using the same. Therefore, combinations of features and methods disclosed herein may not be necessary to practice the disclosure in its broadest sense and are instead disclosed merely to particularly describe representative and preferred embodiments.

Various modifications to the embodiments may be apparent to one of skill in the art upon reading this disclosure. For example, persons of ordinary skill in the relevant arts will recognize that the various features described for the different embodiments can be suitably combined, un-combined, and re-combined with other features, alone, or in different combinations. Likewise, the various features described above should all be regarded as example embodiments, rather than limitations to the scope or spirit of the disclosure.

Persons of ordinary skill in the relevant arts will recognize that various embodiments can comprise fewer features than illustrated in any individual embodiment described above. The embodiments described herein are not meant to be an exhaustive presentation of the ways in which the various features may be combined. Accordingly, the embodiments are not mutually exclusive combinations of features; rather, the claims can comprise a combination of different individual features selected from different individual embodiments, as understood by persons of ordinary skill in the art.

The following references are hereby incorporated by reference herein in their entirety except for patent claims and express definitions contained therein: International Application No. PCT/US19/42491 to Altshuler, et al., filed July 18, 2019 and owned by the owner of the present application; U.S. Patent No. 9,775,675 to Irby, III. Any incorporation by reference of documents herein is limited such that no subject matter is incorporated that is contrary to the explicit disclosure herein.

Unless indicated otherwise, references to "embodiment(s)", "disclosure", "present disclosure", "embodiment(s) of the disclosure", "disclosed embodiment(s)", and the like contained herein refer to the specification (text, including the claims, and figures) of this patent application that are not admitted prior art.

For purposes of interpreting the claims, it is expressly intended that the provisions of 35 U.S.C. 112(f) are not to be invoked unless the specific terms "means for" or "step for" are recited in the respective claim.

### EMBODIMENTS

Although the present invention is defined in the attached claims, it should be understood that the present invention can also (alternatively) be defined in accordance with the following embodiments:
1. An endoscopic surgical instrument, comprising:
   a catheter shaft defining and extending along a central axis and including a proximal portion and a distal portion;
   a distal head portion disposed at said distal portion of said catheter shaft, said distal head portion including a distal face;
   a working channel extending within said catheter shaft from said proximal portion through said distal head portion, said distal head portion defining a mouth at said distal face, said working channel being configured to receive a laser fiber;
   an illuminator disposed at said distal head portion; and
   an imaging receiver disposed at said distal head portion, said imaging receiver being positioned at an axial distance proximal to a distal extremity of said distal face, said axial distance being within a range of 1 millimeter to 10 millimeters inclusive,
   wherein said mouth is at least partially within a viewing angle of said imaging receiver.
2. The endoscopic surgical instrument of embodiment 1, wherein said working channel is defined by and unitary with said catheter shaft.
3. The endoscopic surgical instrument of embodiment 1, comprising a laser fiber for insertion into said working channel.
4. The endoscopic surgical instrument of embodiment 1, wherein said catheter shaft includes a shaft cross-section normal to a central axis of said catheter shaft that defines an oblong shape, said shaft cross-section defining a major axis that passes through a maximum dimension of said oblong shape and a minor axis that is perpendicular to said major axis.
5. The endoscopic surgical instrument of embodiment 4, wherein said oblong shape is an oval.
6. The endoscopic surgical instrument of embodiment 4, wherein said maximum dimension of said shaft cross-section is in a range of 2.2 millimeters to 2.5 millimeters inclusive.
7. The endoscopic surgical instrument of embodiment 4, wherein said minimum dimension of said shaft cross-section is in a range of 1.7 millimeters to 2.0 millimeters inclusive.
8. The endoscopic surgical instrument of embodiment 1, wherein said distal head portion includes a distal tip portion in contact with distal portion of said catheter shaft, said imaging receiver being mounted to said distal tip.
9. The endoscopic surgical instrument of embodiment 8, wherein said distal tip portion includes said distal face.
10. The endoscopic surgical instrument of embodiment 7, wherein said distal tip portion is unitary with said catheter shaft.
11. The endoscopic surgical instrument of embodiment 7, wherein said distal head portion includes a transparent medium distal to and affixed to said distal tip portion, said transparent medium including said distal face.
12. The endoscopic surgical instrument of embodiment 11, wherein said mouth is at least partially visible through said transparent medium via the imaging receiver
13. The endoscopic surgical instrument of any one of embodiments 1 - 12, wherein said working channel is an aspiration channel.
14. The endoscopic surgical instrument of embodiment 1, comprising an irrigation channel in fluid communication with an outlet, said outlet being defined by said distal head.
15. The endoscopic surgical instrument of embodiment 14, wherein said irrigation channel is defined by an internal hollow of said catheter shaft exclusive of said aspiration channel, said internal hollow extending from said proximal portion of said catheter shaft to said distal portion of said catheter shaft.
16. The endoscopic surgical instrument of embodiment 14, wherein said outlet of said irrigation channel is configured at an outlet angle relative to a distal direction along said central axis.
17. The endoscopic surgical instrument of embodiment 16, wherein said distal head portion includes a distal tip portion in contact with said distal portion of said catheter shaft, said outlet being defined by said distal tip portion.
18. The endoscopic surgical instrument of embodiment 17, wherein said outlet angle is in a range of 0 degrees to 170 degrees inclusive.
19. The endoscopic surgical instrument of embodiment 17, wherein said outlet angle is in a range of 10 degrees to 70 degrees inclusive.
20. The endoscopic surgical instrument of embodiment 17, wherein said outlet angle is in a range of 20 degrees to 45 degrees inclusive.
21. The endoscopic surgical instrument of embodiment 16, wherein said distal head portion includes a distal tip portion in contact with said distal portion of said catheter shaft and a transparent medium distal to and affixed to said distal tip portion, said outlet being defined by said distal tip portion and configured to direct irrigation flow onto a proximal face of said transparent medium.
22. The endoscopic surgical instrument of embodiment 1, wherein a distal end of said laser fiber is selectively positionable over a range of axial positions relative to a distal-most location of said mouth.
23. The endoscopic surgical instrument of embodiment 22, wherein said range of axial positions is not greater than 1 millimeter distal to said distal-most location of said mouth and not greater than 3 millimeters proximal to said distal-most location.
24. The endoscopic surgical instrument of embodiment 22, wherein said range of axial positions is from flush with said distal-most location of said mouth and to not greater than 1 millimeter proximal to said distal extremity.
25. The endoscopic surgical instrument of embodiment 22, wherein said range of axial positions is not less than 0.1 millimeter distal to said distal-most location of said distal tip and not greater than 0.6 millimeters proximal to said distal extremity.
26. The endoscopic surgical instrument of embodiment 1, wherein said illuminator is a fiber optic, said fiber optic being anchored to said distal head portion.
27. The endoscopic surgical instrument of embodiment 26, wherein said catheter shaft is flexible with said proximal portion of said catheter shaft coupled to a handle, said handle including a steering mechanism that is coupled to said distal head portion via said fiber optic for manipulation of said distal head portion.
28. An endoscopic surgical instrument, comprising:
   a catheter including a flexible catheter shaft coupled to a distal head;
   a first optical fiber extending through said catheter and into said distal head, said first optical fiber being anchored to said distal head;
   a steering handle coupled to said catheter and said optical fiber, said steering handle being configured to exert forces on said first optical fiber for articulation of said distal head.
29. The endoscopic surgical instrument of embodiment 28, wherein said first optical fiber is anchored to said distal head with an adhesive.
30. The endoscopic surgical instrument of embodiment 28, wherein said first optical fiber defines an oblong cross-section defining a major dimension and a minor dimension, said major dimension being a maximum dimension of said oblong cross-section, said minor dimension being less than said major dimension and perpendicular to said major dimension at a central axis of said catheter.
31. The endoscopic surgical instrument of embodiment 28, comprising a second optical fiber extending through said catheter and into said distal head, said second optical fiber being anchored to said distal head.
32. The endoscopic surgical instrument of embodiment 31, wherein said first optical fiber and said second optical fiber are anchored within said distal head at locations that proximate an outer radial dimension of said distal head and are diametrically opposed about said central axis of said catheter and proximate an outer radial surface of said distal head.
33. The endoscopic surgical instrument of embodiment 32, wherein said first optical fiber is one in a first bundle of optical fibers and said second optical fiber is one in a second bundle of optical fibers.
34. The endoscopic surgical instrument of embodiment 33, wherein each of said first bundle of optical fibers and said second bundle of optical fibers are arranged sequentially in a tangential direction about said central axis of said catheter at said distal head.
35. The endoscopic surgical instrument of embodiment 33, wherein each of said first bundle of optical fibers and said second bundle of optical fibers are centered about a respective plane at said distal head.
36. The endoscopic surgical instrument of embodiment 31, wherein said first optical fiber and said second optical fiber each define an oblong cross-section defining a major dimension and a minor dimension, said major dimension being a maximum dimension of said oblong cross-section, said minor dimension being less than said major dimension and perpendicular to said major dimension at a central axis of said catheter.
37. The endoscopic surgical instrument of embodiment 36, wherein said major dimension is in a range of 0.2 to 2.0 millimeters inclusive.
38. The endoscopic surgical instrument of embodiment 36, wherein said minor diameter is in a range of 0.1 to 1.0 millimeters inclusive.
39. The endoscopic surgical instrument of embodiment 36, wherein a ratio of said major diameter to said minor diameter is in a range of 2:1 and 5:1 inclusive.
40. The endoscopic surgical instrument of any one of embodiments 28 - 39, wherein said steering handle includes a rotating cam directly coupled to said first optical fiber and said second optical fiber.
41. The endoscopic surgical instrument of embodiment 40, wherein:
   said first optical fiber is pulled in tension when said rotating cam is actuated in a first rotational direction to articulate said distal head in a first lateral direction; and
   said second optical fiber is pulled in tension when said rotating cam is actuated in a second rotational direction to articulate said distal head in a second lateral direction.
42. The endoscopic surgical instrument of embodiment 40, wherein said second rotational direction is opposite said first rotational direction.
43. The endoscopic surgical instrument of embodiment 40, wherein said second lateral direction is opposite said first lateral direction.
44. The endoscopic surgical instrument of embodiment 40, wherein said first optical fiber and said second optical fiber are bonded to said rotating cam.
45. The endoscopic surgical instrument of embodiment 40, said rotating cam is coupled to a rotatable shaft.
46. The endoscopic surgical instrument of embodiment 37, wherein said rotating cam is coupled to a thumb lever.
47. The endoscopic surgical instrument of embodiment 40, wherein said first optical fiber and said second optical fiber are operatively coupled to an illumination source and are routed from said illumination source to said rotating cam, and from said rotating cam to said distal head.
48. The endoscopic surgical instrument of embodiment 47, wherein said illumination source is a light emitting diode.
49. The endoscopic surgical instrument of embodiment 47, wherein said illumination source is housed within said steering handle.
50. The endoscopic surgical instrument of embodiment 28, wherein said transparent medium defines a pressure relief that extends from said mouth.
51. The endoscopic surgical instrument of embodiment 50, wherein said pressure relief extends radially to an outer perimeter of said transparent medium.
52. The endoscopic surgical instrument of embodiment 50, wherein said pressure relief extends radially to an outer perimeter of said distal face.
53. The endoscopic surgical instrument of embodiment 28, wherein catheter shaft is flexible.
54. The endoscopic surgical instrument of embodiment 28, wherein a pressure sensor is operatively coupled to said working channel.
55. The endoscopic surgical instrument of any one of embodiments 28 - 54, wherein said optical fiber is configured to deliver visible light to a target zone that is distal to said distal head.
56. An endoscopic surgical instrument for removing body stones from an internal organ, comprising:
   a catheter shaft that defines and extends along a central axis and having a proximal portion coupled to a handle;
   a distal tip portion coupled to a distal portion of said catheter shaft;
   a transparent medium coupled to said distal tip portion and including a distal face;
   a working channel extending through said catheter shaft and said transparent medium from said proximal portion of said catheter shaft through said distal face of said transparent medium;
   an illuminator disposed at said distal tip portion; and
   an imaging receiver disposed at said distal tip and proximal to said transparent medium,
   wherein said distal face of said transparent medium defines a mouth of said working channel and is positioned from said imaging receiver at an axial distance that is in a range of 1 millimeter to 10 millimeters inclusive.
57. The endoscopic surgical instrument of embodiment 56, wherein said mouth of said working channel is positioned at said axial distance that is in a range of 1.2 millimeters to 5 millimeters inclusive.
58. The endoscopic surgical instrument of embodiment 56, wherein said working channel is an aspiration channel.
59. The endoscopic surgical instrument of embodiment 56, wherein said working channel is an irrigation channel.
60. The endoscopic surgical instrument of embodiment 59, wherein said irrigation channel defines at least one outlet at said distal tip for directing irrigation flow at an angle relative to said central axis that is within a range of 0 degrees to170 degrees inclusive.
61. The endoscopic instrument of embodiment 59, wherein said angle is within a range 10 degrees to- 70 degrees inclusive.
62. The endoscopic instrument of embodiment 59, wherein said angle is within a range 20 degrees to- 45 degrees inclusive.
63. The endoscopic surgical instrument of embodiment 56, comprising a laser fiber, a portion of which extends through said catheter shaft.
64. The endoscopic surgical instrument of embodiment 63, wherein said laser fiber is inserted into said working channel.
65. The endoscopic surgical instrument of embodiment 63, wherein said laser fiber is permanently integrated within said catheter shaft.
66. The endoscopic surgical instrument of embodiments 63 through 65, wherein a distal end of said laser fiber is selectively positionable at axial positions ranging from 1 millimeter distal to a distal-most location of said mouth to 3 millimeters proximal to said distal face inclusive.
67. The endoscopic surgical instrument of embodiment 66, wherein said axial positions range from flush with said distal face to 1 millimeter proximal to said distal face inclusive.
68. The endoscopic surgical instrument of embodiment 66, wherein said axial positions range from 0.1 millimeter to 0.6 millimeter inclusive proximal to said distal face.
69. The endoscopic surgical instrument of embodiment 56, wherein a cross-sectional area of said mouth of working channel is in a range of 5% to 50% smaller than a cross-sectional area of said working channel proximal to said mouth.
70. The endoscopic surgical instrument of embodiment 56, wherein said transparent medium defines a pressure relief that extends from said mouth.
71. The endoscopic surgical instrument of embodiment 70, wherein said pressure relief extends radially to an outer perimeter of said transparent medium.
72. The endoscopic surgical instrument of embodiment 70, wherein said pressure relief extends radially to an outer perimeter of said distal face.
73. The endoscopic surgical instrument of embodiment 56, wherein catheter shaft is flexible.
74. The endoscopic surgical instrument of embodiment 56, wherein a pressure sensor is operatively coupled to said working channel.
75. The endoscopic surgical instrument of any one of embodiments 56 - 74, wherein said working channel is defined by and unitary with said catheter shaft.
76. A method for removing body stone material from an internal organ, comprising:
   positioning a distal tip of a catheter assembly proximate a body stone material contained within an internal organ, said distal tip including a distal face that defines a mouth of a working channel of said catheter assembly, said body stone material being distal to said mouth; and
   positioning an imaging receiver proximal to said distal tip at a separation distance between said mouth and said imaging receiver while said distal tip is proximate said body stone material, said separation distance being in a range of 1 millimeter to 10 millimeters inclusive.
77. The method of embodiment 76, wherein said separation distance during the step of positioning an imaging receiver is in a range of 1.2 millimeters to 5 millimeters.
78. The method of embodiment 77, comprising illuminating a target zone that surrounds said stone material with visible light.
79. The method of embodiment 78, comprising obtaining an image of a targeted stone and said target zone using said imaging receiver.
80. The method of embodiment 76, comprising positioning a laser fiber within said working channel, a distal end of said laser fiber being proximate said mouth.
81. The method of embodiment 80, comprising selectively locating said distal end of said laser fiber within a range of distance that is not greater than 3 millimeters proximal to a distal-most location of said mouth and not greater than 1 millimeter distal to said distal-most location of said mouth, said range of distance being parallel to said axis of said working channel at said mouth.
82. The method of embodiment 80, comprising selectively locating said distal end of said laser fiber within a range of distance that is flush with said mouth and not greater than 1 millimeter proximal to said mouth, said range of distance being parallel to said axis of said working channel at said mouth.
83. The method of embodiment 80, comprising selectively locating said distal end of said laser fiber within a range of distance that is not greater than 0.6 millimeters proximal to said mouth and not less than 0.1 millimeter proximal to said mouth, said range of distance being parallel to said axis of said working channel at said mouth.
84. The method of any one of embodiments 80 - 83, comprising ablating said body stone material using said laser fiber.
85. The method of embodiment 84 wherein an average laser power delivered with said laser fiber is in a range of 120 Watts to 200 Watts inclusive.
86. The method of embodiment 85, comprising operating said working channel as an aspiration channel.
87. The method of embodiment 85, comprising removing products of ablation through said working channel.
88. The method of embodiment 76, comprising delivering an irrigation fluid through said distal tip of said catheter.
89. The method of embodiment 88, comprising delivering a flow of said irrigation fluid at a directed angle that is within a range of 0 degrees to 170 degrees inclusive relative to a distal direction along a central axis of said distal tip.
90. The method of embodiment 88, comprising delivering a flow of said irrigation fluid at a directed angle that is within a range of 10 degrees to 70 degrees inclusive relative to a distal direction along a central axis of said distal tip.
91. The method of embodiment 88, comprising delivering a flow of said irrigation fluid at a directed angle that is within a range of 20 degrees to 45 degrees relative to a distal direction along a central axis of said distal tip.
92. The method of any one of embodiments 76 - 91, wherein said working channel is an aspiration channel.
93. A method of for removing body stone material from an internal organ, comprising:
   inserting an endoscopic surgical instrument that includes a catheter shaft defining and extending along a central axis, said catheter shaft including a proximal portion coupled to a handle and a distal tip portion at distal portion , said catheter shaft including an aspiration channel extending from said proximal portion to said distal tip portion with an imaging receiver disposed at said distal tip, said imaging receiver being positioned at an axial position that is in a range from 1 millimeter to 10 millimeters inclusive from a distal face of said distal tip portion, at least one illuminator disposed at said distal tip, a laser fiber disposed in said aspiration channel with a distal end of said laser fiber being extendable to a distance that ranges from 1 millimeter distal to said distal face of said distal tip to 3 millimeters proximal to said distal face, and an irrigation channel defined by an internal void that extends along a length of said catheter shaft, said irrigation channel and having an outlet at said distal tip that is configured to direct irrigation flow at an angle relative to said central axis that is in a range of 0 degrees to 170 degrees inclusive;
   obtaining an image of a targeted stone and surrounding area;
   placing said distal face proximate said body stone material;
   activating an irrigation flow through said irrigation channel;
   activating an aspiration flow through said aspiration channel to remove products of ablation through aspiration channel; and
   activating a laser coupled to said laser fiber to ablate targeted stone material.
94. A method for removing body stone material from an internal organ, comprising:
   providing a catheter assembly;
   providing operating instructions for said catheter assembly on a non-transitory, tangible medium, said operating instructions including:
      positioning a distal tip of a catheter assembly proximate a body stone material contained within an internal organ, said distal tip including a distal face that defines a mouth of a working channel of said catheter assembly, said body stone material being distal to said mouth; and
      positioning an imaging receiver proximal to said distal tip,
   wherein a separation distance between said mouth and said imaging receiver while said distal tip is proximate said body stone material is in a range of 1 millimeter to 10 millimeters inclusive.
95. The method of embodiment 94, wherein said operating instructions include illuminating a target zone that surrounds said stone material with visible light.
96. The method of embodiment 95, wherein said operating instructions include obtaining an image of a targeted stone and said target zone using said imaging receiver.
97. The method of embodiment 94, wherein said operating instructions include positioning a laser fiber within said working channel so that a distal end of said laser fiber is proximate said mouth.
98. The method of embodiment 97, wherein said operating instructions include selectively locating said distal end of said laser fiber within a range of distance that is not greater than 3 millimeters proximal to a distal-most location of said mouth and not greater than 1 millimeter distal to said distal-most location of said mouth, said range of distance being parallel to said axis of said working channel at said mouth.
99. The method of embodiment 97, wherein said operating instructions include selectively locating said distal end of said laser fiber within a range of distance that is flush with said mouth and not greater than 1 millimeter proximal to said mouth, said range of distance being parallel to said axis of said working channel at said mouth.
100. The method of embodiment 97, wherein said operating instructions include selectively locating said distal end of said laser fiber within a range of distance that is not greater than 0.6 millimeters proximal to said mouth and not less than 0.1 millimeter proximal to said mouth, said range of distance being parallel to said axis of said working channel at said mouth.
101. The method of any one of embodiments 94 - 100, wherein said operating instructions include ablating said body stone material using said laser fiber.
102. The method of embodiment 101 wherein said operating instructions include delivering an average laser power in a range of 120 Watts to 200 Watts inclusive.
103. The method of embodiment 101, wherein said operating instructions include removing products of ablation through said working channel.
104. The method of embodiment 94, wherein said operating instructions include delivering an irrigation fluid through said distal tip of said catheter.
105. The method of embodiment 104, wherein said operating instructions include operating said catheter assembly to deliver a flow of said irrigation fluid at a directed angle that is within a range of 0 degrees to 170 degrees inclusive relative to a distal direction along a central axis of said distal tip.
106. The method of embodiment 105, wherein said operating instructions include operating said catheter assembly to deliver a flow of said irrigation fluid at a directed angle that is within a range of 10 degrees to 70 degrees inclusive relative to a distal direction along a central axis of said distal tip.
107. The method of embodiment 105, wherein said operating instructions include operating said catheter assembly to deliver a flow of said irrigation fluid at a directed angle that is within a range of 20 degrees to 45 degrees relative to a distal direction along a central axis of said distal tip.
108. The method of any one of embodiments 93 - 107, wherein said operating instructions include operating said working channel is an aspiration channel.

## Claims

1. An endoscopic surgical instrument, comprising:
a catheter shaft (33) defining and extending along a central axis (110) and including a proximal portion (36) and a distal portion (35);
a distal head portion (34) disposed at said distal portion (35) of said catheter shaft, said distal head portion including a distal face (98);
a working channel (102) extending within said catheter shaft (33) from said proximal portion (36) through said distal head portion (34), said distal head portion defining a mouth (108) at said distal face (98), said working channel being configured to receive a laser fiber (112);
an illuminator (130) disposed at said distal head portion (34); and
an imaging receiver (142) disposed at said distal head portion (34), said imaging receiver being positioned at an axial distance proximal to a distal extremity of said distal face (98), said axial distance being within a range of 1 millimeter to 10 millimeters inclusive, wherein said mouth (108) is at least partially within a viewing angle (148) of said imaging receiver (142).

2. The endoscopic surgical instrument of claim 1, comprising a laser fiber (112) disposed in the working channel.

3. The endoscopic surgical instrument of claim 2, wherein a distal end (114) of the laser fiber (112) is positioned proximate the mouth (108).

4. The endoscopic surgical instrument of claim 2 or 3, wherein a distal end (114) of the laser fiber (112) is recessed relative to a distal face (106) of a transparent cap (100) that is secured to the distal face (98) of the distal head portion (34).

5. The endoscopic surgical instrument of claim 2 or 3, wherein the working channel (102) includes a working port (103) that is formed in and passes through the distal head portion (34) and defines the mouth (108), and
wherein a distal end (114) of the laser fiber (112) is recessed within the working port (103) proximal to a distal face (106) of a transparent cap (100) that is secured to the distal face (98) of the distal head portion (34).

6. The endoscopic surgical instrument of claim 1, wherein the working channel (102) includes a working port (103) that is formed in and passes through the distal head portion (34) and defines said mouth (108),
wherein a laser fiber optic port (266) protrudes radially into the working port (103), and wherein the laser fiber (112) is mounted within the laser fiber optic port (266).

7. The endoscopic surgical instrument of claim 2, wherein a distal end (114) of said laser fiber (112) is selectively positionable over a range of axial positions relative to a distal-most location of said mouth (108).

8. The endoscopic surgical instrument of any one of claims 3 to 7, wherein the distal end (114) of the laser fiber (112) is within the viewing angle (148) of the imaging receiver (142).

9. The endoscopic surgical instrument of any one of claims 1 to 8, wherein the viewing angle of the imaging receiver (142) is within a range of 90 to 120 degrees inclusive.

10. The endoscopic surgical instrument of claim 1, comprising an irrigation channel in fluid communication with an outlet (218), said outlet being defined by said distal head portion.

11. The endoscopic surgical instrument of claim 10, wherein said outlet (218) of said irrigation channel is configured at an outlet angle relative to a distal direction along said central axis (110).

12. The endoscopic surgical instrument of claim 11, wherein the outlet (218) is configured to deliver an irrigation flow (252) that vectors radially outward.

13. The endoscopic surgical instrument of claim 11 or 12, wherein said distal head portion (34) includes a distal tip portion (96) in contact with said distal portion (35) of said catheter shaft, said outlet (218) being defined by said distal tip portion.

14. The endoscopic surgical instrument of claim 11 or 12, wherein said distal head portion (34) includes a distal tip portion (96) in contact with said distal portion (35) of said catheter shaft and a transparent medium distal to and affixed to said distal tip portion, said outlet (218) being defined by said distal tip portion and configured to direct irrigation flow (252) onto a proximal face of said transparent medium.

15. The endoscopic surgical instrument of any one of claims 10 to 12, wherein said working channel (102) is an aspiration channel, wherein said irrigation channel is defined by an internal hollow of said catheter shaft (33) exclusive of said aspiration channel, said internal hollow extending from said proximal portion (36) of said catheter shaft to said distal portion (35) of said catheter shaft.

16. The endoscopic surgical instrument of any one of claims 1 to 15, wherein said working channel (102) is an aspiration channel, and wherein an aspiration flow (254) draws flow into the mouth (108) at said distal face (98) of the distal head portion (34).
